# EUROPEAN PATENT SPECIFICATION

(11) **EP 1 850 889 B1**
(45) Date of publication and mention of the grant of the patent: **05.05.2010**
(21) Application number: 06711168.2
(22) Date of filing: 14.02.2006
(51) Int. Cl.: A61L 15/60, A61K 8/73, A61K 9/00, A61Q 19/00

(54) **DEPOLYMERIZED POLYSACCHARIDE-BASED HYDROGEL ADHESIVE AND METHODS OF USE THEREOF**
HYDROGEL-KLEBSTOFFE AUF BASIS VON DEPOLYMERISIERTEN POLYSACCHARIDEN UND VERFAHREN ZU DESSEN VERWENDUNG
HYDROGEL ADHESIF DEPOLYMERISE A BASE DE POLYSACCHARIDE, ET SON PROCEDE D'UTILISATION

(30) Priority: 14.02.2005 US 652816 P
(43) Date of publication of application: 07.11.2007
(73) Proprietor: Yissum Research Development Company, of The Hebrew University of Jerusalem, 91390 Jerusalem (IL)
(72) Inventor: NUSSINOVITCH, Amos, 76566 Rehovot (IL); BEN-ZION, Omri, 47224 Ramat Hasharon (IL)
(74) Representative: Becker Kurig Straus
(86) International application number: PCT/IL2006/000186
(87) International publication number: WO 2006/085329

(56) References cited:
- WO-A-02/06373
- GB-A- 2 362 100
- US-A- 3 640 741
- US-A- 4 299 231
- US-A- 5 536 263
- US-A- 2004 083 819

## Description

### FIELD OF THE INVENTION

The present invention provides hydrogel adhesives based on chemically and physically modified polysaccharides, which are partially depolymerized, useful for wound healing and topical and transdermal delivery of therapeutic and cosmetic agents and methods of preparation thereof. The present invention further provides methods and a device useful for the testing the adhesive properties of hydrogels.

### BACKGROUND OF THE INVENTION

### Pressure Sensitive Adhesives

Pressure-sensitive adhesives (PSAs) are adhesives that are capable of bonding to surfaces via brief contact under light pressure (Goulding, 1994). PSA's are an indispensable component of medicinal patches, medical devices, tapes, dressings and bioelectrodes. Several basic requirements must be fulfilled to provide an acceptable PSA product including (1) adequate skin adhesion and cohesion; (2) biocompatibility i.e. biologically inert, precluding contact dermatitis, allergy, sensitivity or toxicity; (3) repositioning ability on the skin surface for multiple applications; (4) small geometric dimensions; (5) reasonable cost; and (6) compliance with international pharmaceutical standards.

Elastomers are flexible polymer materials that function to increase the elasticity, tear resistance, and cohesiveness of adhesive compositions. Many of the known PSA elastomers cause physiological irritation including inflammation of sweat glands, keratin peeling, tissue injury after adhesive removal and contact dermatitis due to prolonged contact with the skin (Bergman et al., 1982; Hammond, 1989).

Three types of polymers are commonly used in PSA dermatological products, particularly transdermal delivery (TDD) systems: polyisobutylenes (PIB), polysiloxanes (silicones) and polyacrylate copolymers (Tan and Pfister, 1999). These polymers have several notable disadvantages. First, they are hydrophobic and retain only a small amount of moisture (<0.1 %) after drying, thus limiting the type of active agents that can be incorporated and diminishing the electrical conductivity potential in iontophoresis. Moreover, the hydrophobic nature of the PSA prevents wick removal of accumulated moisture on the skin surface, increasing the risk of microbial infection. In addition, they are typically rigid, becoming soft and flexible only when their temperature exceeds the glass transition, posing problems in industrial manufacturing.

### Hydrocolloids and Hydrogels

The art recognizes medicinal polymeric hydrocolloidal materials that are mucoadhesive, i.e. adhere to a subject's mucous membranes. In such applications, the dried hydrocolloids are applied to the mucosal tissue and tack occurs by swelling of the polymer by the biological fluids. Different chemo-physical factors affect mucoadhesion properties including type of polymer, its concentration and molecular weight (Chen and Cyr, 1970); viscosity of the polymer dispersion; matrix hydration capability; polymeric mixtures; polymer pH and electrical charge; adhesive-layer thickness; and shearing (Chen and Cyr, 1970).

*Sterculia* gum, also known as gum karaya, is a hydrophilic colloid prepared from the exudate of the *Sterculia Urens* tree. It is a complex polysaccharide gum comprised mainly of D-galacturonic acid, D-galactose and L-rhamnose, having a molecular weight of about 9-10 × 10⁶ Daltons.

US Patent No. 4,299,231 (herein "'231") discloses an electrically conductive, visco-elastic gel comprising 10 to 50% of a high molecular weight polysaccharide such as karaya gum, 90 to 20% of at least one polyol, the polyol having a water content of 5 to 20% by weight, 0 to 30% of at least one non-volatile acid soluble in said polyol, 0 to 30% of at least one non-volatile base soluble in said polyol for use in adhering or producing medical electrodes. The preferred polysaccharides include gum karaya, gum tragacanth, xanthan gum, and carboxymethylcellulose. The gels are disclosed as having relatively low water content, which allows open-air storage. Modified depolymerized polysaccharides and use of the gels for therapeutic and cosmetic indications are neither taught nor suggested. In fact, '231 teaches away from the use of depolymerized polysaccharides by stating that gum karaya yields the best results, probably because of its high molecular weight of about 9.5 × 10⁶ Daltons.

US Patent. No. 3,640,741 (herein "'741") teaches a mixture of a hydrophilic gum and a cross-linking agent, such as propylene glycol, in a non water-soluble carrier, the mixture forming a gel, useful for providing for timed release of medication in the body or cosmetic additives on the surface of a person's skin. In one specific embodiment the hydrophilic gum comprises a mixture of carboxymethylcellulose or sodium alginate and karaya gum. According to that disclosure, karaya gum should not be used to fully substitute the cellulose or alginate gums. Modified polysaccharides are neither taught nor suggested in '741.

US Patent No. 4,306,551 (herein "'551") teaches a flexible, liquid absorbable adhesive bandage comprising a backing and a substrate, the substrate comprising a solid phase comprising 30%-50% by weight and a liquid phase of hydric alcohol, carbohydrates or proteins comprising 50-70% by weight, further comprising a synthetic resin selected from polyacrylic acid, polyacrylamide and their cogeners. In certain embodiments of that patent, synthetic polymers or natural polysaccharide gums constitute the solid phase. In certain embodiments the natural gum is karaya gum. That disclosure teaches that a synthetic resin is needed in forming a matrix based on karaya gum in order to protect the karaya during gamma radiation sterilization. In certain embodiments a salt replaces the synthetic resin. US Patent No. 4,307,717 teaches a flexible, liquid-absorbent, adhesive bandage comprising the matrix taught in the '551 patent, further comprising a medicament for release to the surface to which the bandage is applied. The above patents neither teach nor suggest advantages of depolymerization of the polysaccharides.

US Patent No. 4,778,786 teaches a gelation reaction product of a mixture of an organic polysaccharide gum, polyethylene glycol, and m-, p- or o-hydroxybenzoic acid in an amount effective in forming a gel having adhesive properties for adhesion to skin for transdermal drug delivery. The '786 patent teaches that polyethylene glycol and m-, p- or o-hydroxybenzoic acid combine with polysaccharide gums to form a gel having both desirable tackiness/deformability and desirable structural integrity whereas polyethylene glycol and polysaccharide gums, without m-, p- or o-hydroxybenzoic acid, mostly fail to form gels or form mushy gels lacking structural integrity even at modest concentrations of polyethylene glycol.

US Patents No. 5,536,263 and 5,741,510 teach a non-occlusive medication patch to be applied to the skin, the patch comprising a porous backing and a flexible hydrophilic pressure-sensitive adhesive reservoir comprising a hydrocolloidal gel for the sustained release of medication through the skin of a patient. The reservoir has two portions: an external coating layer with an exposed lower skin-contacting surface that forms a pressure-sensitive bond with the skin, and an upper internal portion which infiltrates the porous backing and becomes solidified therein after being applied so that the reservoir and the backing are unified, enabling the backing itself to act as a storage location for the medication-containing reservoir.

There remains a yet unmet need for a pressure-sensitive adhesive (PSA) useful in pharmaceutical and cosmetic applications. The art has neither taught nor suggested a nonocclusive hydrogel adhesive comprising a physically- or chemically-depolymerized polysaccharide.

### SUMMARY OF THE INVENTION

The present invention discloses hydrophilic compositions comprising polysaccharide-based hydrocolloid gum exudates modified by chemical or physical means to provide superior pressure sensitive adhesive (PSA) materials. The simplicity of the matrix, derived from its preparation methods, and ease of manufacture, provides a significant advantage over standard PSA materials. It is further disclosed that these modified polysaccharide-based hydrogels are particularly useful as depots for biologically active ingredients for pharmaceutical or cosmeceutic use.

The inventors have unexpectedly found that hydrogels produced from polysaccharides which are partially depolymerized exhibit cohesiveness and adhesiveness and are particularly useful as pressure sensitive adhesives (PSA) in medicinal and cosmetic applications. Depolymerization may be carried out by chemical and physical techniques including gamma irradiation, a combination of ozone and UV radiation and sonication. Partial depolymerization of the polysaccharides is carried out by controlled physical and or chemical means, thereby providing a product that is both tacky and cohesive.

The inventors have also found that despite the hitherto known observation that addition of alkali to increase the pH of acid acetylated polysaccharides in solvent-non-solvent hydrogels results in a soft, non-tacky product, the addition of borate or a volatile acid provides cohesive and adhesive gels having a broad and useful pH range.

Accordingly, in one aspect, the present invention provides a bioadhesive hydrogel pharmaceutical composition comprising
a) 10% to 50% w/w of at least one modified hydrophilic polysaccharide;
b) 20% to 50% w/w of at least one non-solvent of the polysaccharide;
c) 10% to 40% w/w of at least one solvent of said polysaccharide; and
d) 10% to 40% w/w of at least one humectant;
wherein the modified hydrophilic polysaccharide is selected from a partially depolymerized polysaccharide, a borate ion cross-linked polysaccharide and an acid cross-linked polysaccharide.

In one embodiment the composition comprises 20% to 30% w/w of at least one modified hydrophilic polysaccharide. In some embodiments the composition comprises 25% to 35% w/w of at least one non-solvent of the polysaccharide. In some embodiments the composition comprises 15% to 25% w/w of at least one solvent of the polysaccharide; and in some embodiments the composition comprises 20% to 30% w/w of at least one humectant.

The components of the composition are provided as percent weight per total weight (% w/w).

The composition can be used *per se* or in combination with therapeutic or cosmetic agents. Accordingly, in some embodiments the composition further comprises a therapeutically effective amount of one or more pharmaceutically active agents. In other embodiments the composition further comprises one or more cosmetic agents.

In one embodiment the modified hydrophilic polysaccharide is a partially depolymerized hydrophilic polysaccharide. In some embodiments the modified polysaccharide has a molecular weight average of 0.1 x 10⁶ to 9 x 10⁶ Daltons, preferably from 0.2 x 10⁶ to 5 x 10⁶ Daltons. A partially depolymerized polysaccharide refers to a polysaccharide having a reduced molecular weight when compared to the native polysaccharide.

The polysaccharide undergoes partial depolymerization using at least one method selected from gamma irradiation, UV radiation, ozone exposure, sonication, mechanical pressure, heating or acid hydrolysis. In specific embodiments the polysaccharide is partially depolymerized by gamma irradiation. In another embodiment the polysaccharide is partially depolymerized by exposure to ozone and UV radiation.

The polysaccharide is selected to impart excellent adhesive and cohesive properties to the gel prepared with a partially depolymerized polysaccharide. Preferably the polysaccharide is a heteropolysaccharide. In some embodiments the polysaccharide is selected from a polysaccharide exudate that derives from the *Sterculiaceae* and *Leguminosea* (*Fabaceae*) families of herbs, shrubs and trees. In one embodiment the polysaccharide is *Sterculia urens* (*S. urens;* gum karaya). In another embodiment the polysaccharide is *Acacia senegal* (gum arabic).

In some embodiments the polysaccharide is mixture of two or more polysaccharides. The mixture comprises two fractions having different molecular weight averages, one fraction referred to as a high molecular weight (HMW) fraction and a second fraction referred to as a low molecular weight (LMW) fraction. The HMW fraction comprises at least one polysaccharide having a molecular weight in the range of 2 x 10⁶ to 9 x 10⁶ Daltons and the low molecular weight fraction comprises at least one polysaccharide having a molecular weight in the range of 0.1 x 10⁶ to 2 x 10⁶ Dalton. In specific embodiments the hydrogel comprises a blend of HMW and LMW fractions of gum karaya. The proportion of the polysaccharides in a composition is 10% to 90% of the HMW polysaccharide and 90% to 10% of the LMW polysaccharide. The ratio of HMW polysaccharides to LMW polysaccharides is 9:1 to 1:9. In specific embodiments the ratio is 1:1.

In other embodiments the modified polysaccharide is a borate ion crosslinked polysaccharide. In specific embodiments the source of borate ions is selected from boric acid and sodium tetraborate decahydrate. In some embodiments the source of borate ions is boric acid. In one embodiment the source of borate ions is provided at a final concentration of 0.5% to 5% (w/w).

In some embodiment the modified polysaccharide is an acid treated polysaccharide which has been treated with a volatile acid. In preferred embodiments the acid is hydrochloric acid (HCl). In one embodiment the acid is provided at a final concentration of 0.5% to 5% (w/w).

In certain embodiments the composition of the present invention further comprises a base. In specific embodiments the composition of the present invention comprises boric acid together with a base, or sodium tetraborate decahydrate. In some embodiments the base is selected from sodium hydroxide and potassium hydroxide to a final concentration of 0.3% to 15% (w/w).

The borate ion and acid modified hydrogel compositions have a pH ranging from pH 2 to pH 11. The pH of the composition will be selected based on several factors including the method of use and the addition of any bioactive or therapeutic agents.

An appropriate polysaccharide for modification by borate ion and acid treatment is a polysaccharide exudate selected from the group consisting of *Sterculiaceae, Cochlospermaceae, Rutaceae, Proteaceae, Combretaceae, Rosaceae, Anacardiaceae, Meliaceae, Leguminosae, Rhizophoraceae, Celastraceae, Moringaceae, Sapindaceae, Annonaceae, Burseraceae, Bombacaceae, Arecaceae, Lythraceae, Lecythidaceae, Boraginaceae, Malvaceae, Cactaceae, Bromeliaceae, Guttiferae Sapotaceae, Pinaceae, Capparidaceae, Araliaceae, Vitaceae, Penaeaceae, Zamiaceae, Cycadaceae, Stangeriaceae, Ebenaceae, Agavaceae, Elaeocarpaceae, Cunoniaceae, Gymnospermeae, Ochnaceae, Rhamnaceae, Euphorbiaceae* and *Pittosporaceae* families of herbs, shrubs and trees. In specific embodiments the polysaccharide exudate is selected from *Sterculia urens* and *Bauhinia variegata* species.

In certain embodiments the modified polysaccharide has been modified by one or more treatments. In some embodiments the polysaccharide is a borate ion cross-linked, partially depolymerized polysaccharide.

A non-solvent is a liquid in which the polysaccharide is non-soluble and is able to disperse. Preferred non-solvents for the polysaccharide are polyhydric alcohols, including in a non-limiting manner propylene glycol, dipropylene glycol, polyethylene glycol, butylene glycol, hexylene glycol, polyoxyethylene glycol, polypropylene glycol and ethylene glycol. In certain embodiments the non-solvent of the present invention is propylene glycol. The composition comprises 20% to 50% (w/w) non-solvent of the polysaccharide. In some embodiments the composition comprises 25% to 35% (w/w) non-solvent.

The solvent is selected as a solvent of the polysaccharide and is present in the composition at a concentration of 10% to 40% (w/w). In some embodiments the concentration of solvent is 15% to 25% w/w. In specific embodiments the solvent is water.

The humectant according to the present invention is selected from the group consisting of glycerol, sorbitol and maltitol. In specific embodiments the humectant is glycerol. The humectant is present in the composition at a concentration of 10% to 40% (w/w). In specific embodiments the composition comprises 20% to 30% (w/w) glycerol.

In one embodiment the composition comprises a backing layer.

In a second aspect, the present invention provides a method of preparing an adhesive hydrogel pharmaceutical composition comprising 10% to 50% w/w of at least one modified hydrophilic polysaccharide; 20% to 50% w/w of at least one non-solvent of the polysaccharide; 10% to 40% w/w of at least one solvent of said polysaccharide; and 10% to 40% w/w of at least one humectant. In one embodiment the method comprises the steps of:
a) exposing the polysaccharide to a modifying agent;
b) dispersing said the at least one polysaccharide in at least one non-solvent of said polysaccharide;
c) admixing said solvent with the at least one humectant;
d) combining said polysaccharide-non-solvent dispersion with said solvent-humectant mixture to generate a viscous mixture; and
e) dispensing said viscous mixture.

As one of ordinary skill in the art would appreciate, other sequences of steps may be possible. For example, the polysaccharide may be modified at any of the steps of the method. Therefore, the particular order of the steps set forth in the specification should not be construed as limitations. In certain embodiments the modification of the polysaccharide may precede or follow its dispersion in the non-solvent. In one embodiment the polysaccharide is modified by ozone and UV irradiation following dispensing of the viscous mixture. In another embodiment a source of borate ions or a volatile acid is added to the viscous mixture.

The polysaccharide may be partially depolymerized using at least one method selected from gamma irradiation, UV radiation, ozone exposure, sonication, mechanical pressure, heating, or acid hydrolysis. In specific embodiments the polysaccharide is partially depolymerized by gamma irradiation. In one embodiment the polysaccharide has been depolymerized by ozone and UV radiation.

In other embodiments the polysaccharide is a crosslinked polysaccharide. In certain embodiments the composition further comprises a source of borate ions selected from boric acid and sodium tetraborate decahydrate. In other embodiments the composition further comprises a volatile acid. In some embodiments the source of borate ions is boric acid together with a base. In some embodiments the volatile acid is hydrochloric acid.

A third aspect provides a method of using the hydrogels of the present invention. The pharmaceutical hydrogel compositions of this invention can be used as adhesive patches, wound dressings, bioelectrodes, as a cosmetic agent depot and as a therapeutic agent depot in systems for topical dermal delivery, passive transdermal drug delivery and iontophoretic drug delivery.

Another aspect of the present invention provides a method of treating a subject in need thereof, the method comprising the step of:
topically applying to a part of the body of said subject a bioadhesive composition comprising 10% to 50% w/w of at least one modified hydrophilic polysaccharide; 20% to 50% w/w of at least one non-solvent of the polysaccharide; 10% to 40% w/w of at least one solvent of said polysaccharide; and 10% to 40% w/w of at least one humectant;
wherein the modified hydrophilic polysaccharide is selected from a partially depolymerized polysaccharide, a borate ion cross-linked polysaccharide and an acid crosslinked polysaccharide.

The bioadhesive composition may further comprise a pharmaceutically active or cosmetically active agent.

The composition of the present invention may be applied to any part of the body, to which application of the composition is needed or desired. The composition is useful for both therapeutic and cosmetic applications.

### BRIEF DESCRIPTION OF THE FIGURES

Figure 1 shows the probe tack energy and yield stress of hydrogels composed of various amounts of water and native (not modified) polysaccharide. (probe tack energy: Figure 1A; yield stress: Figure 1B).
Figure 2 shows a master curve for probe tack energy vs. weight average molecular of *S*. *urens* based hydrogels treated by gamma irradiation, mechanical pressure, sonication, heating or acid hydrolysis. Note that the tack energy falls after the peak as MW increases.
Figure 3 depicts the properties of *S. urens* polysaccharide-based hydrogels depolymerized by gamma irradiation. The parameters tested included: probe tack energy (aluminum probe, diameter: 22 mm; bonding: 0.5 N; 70 mm/min; dwell-time: 60s; debonding: 600 mm/min); tangent δ; residue left on the probe after debonding (adhesive failure); crossover point; cohesion calculated from 3-parameter power law model; non-recoverable strain after relaxation (calculated from creep-recovery curves).
Figure 4 shows probe tack and tangent delta of hydrogels composed of MWₐ (3 ×10⁵ g mol⁻¹) and MW_{b} (7.7 × 10⁶ g mol⁻¹) blends of *S. urens* polysaccharides.
Figure 5 shows a graph representing the results of a repositioning simulation by cycling test for hydrogels prepared by modification type I.
Figure 6 is a graph depicting probe tack energy vs. O₃-UV radiation exposure time of the type I modified hydrogels.
Figure 7 is a schematic illustration of a O₃-UV radiation treated type I hydrogel. A: Exposed modified layer with lower molecular weight and improved hydrophilic characteristics. B: hydrogel bulk, having similar properties to the native non-modified hydrogel.
Figure 8 shows graphs depicting various parameters of the type II modified hydrogels including probe tack energy, rheological and molecular parameters correlated to pH.
Figure 9 shows the dwell-time required for complete bond formation for hydrogels of type II tested with aluminum and PTFE (polytetrafluoroethylene) probes.
Figure 10 shows the longitudinal force vs. rolling velocity (10A) and bonding dwell-time (10B) of the hydrogels tested with a rolling-tack apparatus. Probes and hydrogels are same as in Figure 9: longitudinal load: 0.4N/m.
Figure 11A Rolling tack apparatus described elsewhere (Ben-Zion and Nussinovitch, 2002a,b, Nussinovitch, 2000). Figure 11B illustrates the probe-tack device of the present invention
Figure 12 shows a typical illustration curve demonstrating the operation modes of the probe-tack test. The curve shows the time periods for compression to a predetermined load followed by sample relaxation and debonding. The dwell-time can be calculated from initial contact to initial debonding.
Figure 13 is a graph depicting the weight loss of hydrogels prepared via modification type II following 2 months in a non-sealed packaging.

### DETAILED DESCRIPTION OF THE INVENTION

The present invention discloses hydrophilic compositions comprising polysaccharide-based hydrocolloid gum exudates modified by chemical or physical means to provide superior pressure sensitive adhesive (PSA) materials. These hydrogels are particularly useful as depots for biologically active ingredients for pharmaceutical or cosmeceutic use.

### Advantages of the Composition

The hydrogel compositions of the present invention have unique features that provide a number of design advantages, benefiting health providers and patients. These advantages include:
a) cohesiveness for excellent flexibility, strength and integrity;
b) adhesive integrity for prolonged adhesion for single application or multiple repositioning;
c) increased contact with the skin surface to improve adhesion and extent of delivery in TDD (Transdermal Drug Delivery) applications;
d) improved electrical conductivity for iontophoresis;
e) physiological biocompatibility: non-toxic, non-irritant;
f) user-friendly for trouble-free application and painless removal;
g) adhesive failure for painless and complete removal without residue;
h) nonocclusive properties to eliminate microbial contamination;
i) compatibility with a wide range of hydrophilic and hydrophobic drugs at various pHs;
j) compliance with international pharmaceutical standards and regulations;
k) esthetic appearance;
l) long shelf life;
m) simple and cost-effective manufacturing process.

The adhesive patches of the present invention comprising modified polysaccharide gum exudates provide the above benefits. The advantageous properties of the matrices, due to the unique composition, provide a significant advantage over standard PSA materials.

Adhesive failure in the present invention refers to residues left on the surface to which the adhesive is bound, after removal.

The modified hydrogels disclosed in this invention are based on novel modified polysaccharide materials and compositions. The use of modified raw materials or end product provides improved adhesion performance over other known native polysaccharide-based hydrogels.

The hydrogels of this invention are biocompatible and have remarkably predictable PSA properties in addition to optimal adhesiveness and cohesiveness (excellent repositioning capability, prolonged tack and painless peeling). Their hydrophilic characteristics enable their use for drug-n-adhesive (DIA) applications, i.e. they provide the basic medium for entrapment of multi-drug components and materials for cosmetic treatments. The unique viscoelastic properties of the hydrogels provide unusually large contact area with the skin surface leading to potential increased absorption of many active agents. Moreover, being a nonocclusive gel, i.e., enables moisture vapor on the surface of the skin to evaporate through the hydrogel, it prevents the undesirable accumulation of water conductive to bacterial growth. The ability of the hydrophilic gels to absorb water from moist or sweaty skin also prolongs the duration of adhesion. In addition, the presence of water in the gels facilitates electrical conductivity, utilized in iontophoresis. The hydrogels are biologically inert, causing no skin allergies, irritation, sensitivity, or toxicity.

The hydrogels can be produced in a wide range of pH, i.e., 2-12 to accommodate a variety of active ingredients and to suit different dermatological conditions. They can be produced using conventional coaters or casting methods and, as opposed to other common PSAs such as those based on PIB, silicone and polyacrylate, no heat is necessary for drying, an operation usually leading to undesirable chemical reactions and loss of active substances due to evaporation (Benedek and Heymans, 1997).

The hydrogels can be utilized in medical and cosmetic fields such as transdermal drug delivery, medical dressings, bioelectrodes, topical cosmetics or other applications relating to skin.

### Definitions

For convenience and clarity certain terms employed in the specification, examples and claims are described herein.

It is noted that, as used in this specification and the appended claims, the singular forms "a," "an" and "the" include plural referents unless the context clearly dictates otherwise. Thus, for example, "a pharmaceutically active agent" refers not only to a single active agent but also to a mixture of two or more active agents, and the like.

The term "tangent delta" or "tangent δ" refers to loss modulus (G") divided by the storage modulus (G'), i.e., G'/G". Tangent δ is a measure of the relationship between the elastic and viscous natures of the material. The tangent delta is calculated from Dynamic Rheology instruments with an oscillation mode at a predefined frequency and torque.

The term "probe tack energy" refers to the energy measured during separating a Pressure-Sensitive Adhesive and a specific probe in a "probe tack test" typically performed at a very short contact time and low pressure.

As used herein, the term "wound" includes any injury to any portion of the body of a subject including, but not limited to, thermal burns, chemical burns, radiation burns, sunburn; damage to the dermis including injuries sustained during medical procedures such as episiotomies and post-surgical injuries; trauma-induced injuries including cuts, abrasions, bullet and knife wounds; chronic conditions such as pressure sores, bedsores, conditions related to diabetes and poor circulation, and acne.

The terms "reduce scarring" includes preventing or decreasing excess scar formation such as keloids and hypertrophic scars, as well decreasing the extent of scar tissue formation. The compositions and methods of the present invention may also be used cosmetically, to prevent excess scar formation and to treat acne.

The terms "electrotransport" and "iontophoresis" as used herein refer to the transdermal delivery of pharmaceutically active agents by means of an applied electromotive force to an agent-containing depot.

The term "pharmaceutically active agent" or "therapeutic agent" or "drug" as used herein is meant any chemical or biological material or compound which induces a desired local or systemic effect, and is capable of being delivered by passive diffusion or by iontophoresis. Examples of such substances will be set forth below.

The term "transdermal" drug delivery refers to administration of an agent to the skin surface of a subject so that the drug passes through the skin tissue and into the subject's blood stream, thereby providing a systemic effect.

The term "topical administration" or "topical application" is used to mean adhesion of the hydrogel to the skin. It may further include delivery of a pharmaceutically active agent to the skin, as in, for example, the treatment of various skin disorders, including wounds. Topical administration of a pharmaceutically active agent, in contrast to transdermal administration, provides a local rather than a systemic effect.

The term "bioadhesive" as used herein refers to materials that adhere to a surface such as skin without the need for wetting or hydration. A "mucoadhesive" is capable of adhering to mucosal tissue such as the throat, mouth, nasal passage, urethra and rectum.

### Adhesive hydrogels composition and methods of preparation

Three types of modified polysaccharides are disclosed herein and have been classified as Type I or Type II, or a combination of Type I and II, for convenience. A type I modification involves the partial depolymerization of high molecular weight polysaccharides to a lower molecular weight while Type II modified polysaccharides are crosslinked by borate ions or a volatile acid. The combination Type I and Type II are polysaccharides which have been chemically or physically depolymerized and crosslinked.

The formulations require a balance between cohesiveness and tack. A too high crosslink density, resulting from a molar mass greater than 9 million Dalton reduces tackiness of the hydrogel. A too low crosslink density yields a hydrogel, which is difficult to handle and is of little practical value. Such a hydrogel adheres but would, upon removal, leave residues on the surface to which it is affixed since the adhesive characteristics are greater than the cohesive ones.

### Type I Modified Polysaccharide-based Hydrogels: partially depolymerized polysaccharides or polysaccharide combinations

The viscoelastic solid hydrogel consists of structured entanglement of polysaccharide molecules solvated in aqueous alcoholic mixture to a high degree of self association. The nature of molecular size, molecular weight distribution and functional groups, determines the wettability, mechanical interlocking, the dissipated energy during debonding and failure mode of the preparation (i.e., adhesive or cohesive).

Without wishing to be bound to theory, a hydrogel prepared from a partially depolymerized polysaccharide, i.e. a polysaccharide treated to reduce its molecular weight to 0.1 × 10⁶ to 9 × 10⁶ or a mixture of polysaccharides achieved by combining low and high molecular weight fractions provides excellent wetting and an increase in dissipated debonding energy.. The high degree of association capability ensures the integrity of the network and formation of adhesive type failure.

Molecular chain scission of the polysaccharides can be achieved by several methods, e.g., direct gamma irradiation to the polysaccharide powder; or dispersion in water followed by, mechanical pressure, heating or sonication and preferably by irradiation. Prepared hydrogel films can also be exposed to gamma irradiation or UV-Ozone chamber; strong volatile acid such as hydrochloric acid can also be incorporated into the hydrogel to cause acid hydrolysis, which significantly reduces the molar mass. Alternatively, high and low molecular weight combinations can be attained by blending a high molecular weight polysaccharide such as *S. urens* together with swellable arabinogalactan type polysaccharide such as the gum of *Bauhinia variegata* or any acetylated type of gum exudates, excluding the soluble types.

In some embodiments depolymerization is performed at a dose of 1-70 kGy. An average molecular weight of the polysaccharides of 0.1 × 10⁶ to 9 × 10⁶ Daltons is preferred. In specific embodiments the weight average molecular weight of the partially depolymerized polysaccharide is 0.2 × 10⁶ to 8 × 10⁶ Daltons, preferably 1 × 10⁶ to 5 × 10⁶ Daltons. A single polysaccharide or a combination of two or more types of polysaccharides may be used.

The optimal concentration of the polysaccharide, water or nonsolvents of any given molar mass, molar mass combinations, or polysaccharide combinations may be determined by response surface methodology. In general, it is shown that use of maximum concentration of native polysaccharide and minimum concentration of water (to the limit of coating viscosity) yield maximum probe tack while increasing rigidity and deformability to acceptable values. The compositions prepared according to this method generally have a pH ranging from pH 4.5 to pH 6.

### Type II Modified Polysaccharide-based Hydrogels: cross-linked polysaccharide hydrogels with a wide range of pH

Addition of alkali to increase the pH of acidic acetylated polysaccharides in solvent-non-solvent hydrogel form, as described here, usually results in soft non-tacky product. The addition of alkali to increase the pH above 7 in such formulations leads to breakage of hydrogen bonds responsible for self-associations and cohesion of the matrix. The inventors of the present invention have now unexpectedly found that addition of boric acid and sodium hydroxide, which serve as a source of borate ions, provides cohesive and adhesive gels having a broad and useful pH range.

Without wishing to be bound to theory the boric acid-base combination promotes the tetra functional cross-linking of hydroxyls by borate ions and rules the surface pH of the resulting hydrogels. During a short aging time full deacetylation of the starting polysaccharide is reached and the product of sodium acetate provides hydration in non-dissociated form at equilibrium state. The role of the reaction product is to expand the three dimensional network and to reverse the effect of cross-linking formed both by the auxiliary and self hydrogen bonding due to absent of acetyl content which was found to be responsible for conformational expanding by hydration. Formation of sodium acetate prevents syneresis. It is also participating in regulating of the pH. The resulted hydrogels posses both high tack and high dissipated energy during debonding and a pH ranging from pH 2 to pH 11. In specific embodiments the pH is in the range of pH 4 to pH 10.

In other embodiments an excellent polysaccharide gel can also be attained by addition of a volatile acid, including hydrochloric acid, to 0.5 to 5 M (calculated for the water portion) to the composition. The amount or concentration of the acid will determined the balance of adhesive to cohesive properties. Without wishing to be bound to theory the hydrochloric acid increases self-associations by hydrogen bonding.

Any gum of the *Sterculiaceae* family can be employed in this method (namely: *Streculia urens Roxb.; Sterculia villosa Roxb.; Sterculia campanulata Wall.; Sterculia foetida L.; Sterculia guttata Roxb.; Sterculia ornate Wall.; Sterculia Setigera Del.; Sterculia barteri Mast.; Sterculia cinerea A. Rich; Sterculia tragacantha Lindl.; Sterculia cordifolia; Sterculia apetala (Jacq.) H. Karsten.; Brachychiton populneus R. Br.; Firmiana simplex (L.) W. Wight; Sterculia quadrifida R. Br.; Brachychiton rupestris Mitch. ex Lindl.; Streculia hypochra Pierre; Sterculia thorelii Pierre; Sterculia scaphigera Wall; or Theobroma cacao L; Heritiera littoralis Dry. ex. Alit.; Tarrietia argyrodendron Bth.; Hildegardia barteri (Mast.) Kosterm.; Cola cordifolia (Cav.) R. Br.;* or *Cochlospernaum gossypium (L.) DC of the Cochlospermaceae* family.

The compositions of the present invention comprise one or more polysaccharide exudates such as *Acacia* gums or any other arabinogalactan type polysaccharide, which under normal conditions of formulations will not gel due to high solubility in water. Examples are the gums of the *Rutaceae* [*Aegle marmelos (L.) Corr. Serr.; Feronia limonia (L.) Swingle; Chloroxylon swietenia DC.; Citrus spp.; Fagara xanthoxyloides Lam.; Balsamocitrus dawei Stapf; Flindersia maculosa; Geijera mulleri Bth.; Bosistoa sapindiformis F.v. M.; Melicope neurococca Bth.; Pentaceras australis Hk. f]; Proteaceae [Hakea gibbosa (Sm.) Cav.; Grevillea robusta A. Cunn.; Brabeium stellatifolium L]; Combretaceae [Combretum spp.; Anogeissus latifolia Wall.; Anogeissus leiocarpus; Laguncularia racemosa (L.) Gaertn. f.; Terminalia L. spp.]; Rosaceae [Prunus amygdalus Batsch; Prumus avium L.]; Anacardiaceae [Buchanania lanzan Sprengel; Semecarpus Anacardium L. fil.; Anacardium occidentale L.; Spondias spp.; Sclerocarya birrea subsp. caffra, Botswana; Lannea coromandelica (Houtt.) Merrill,]; Meliaceae [Toona ciliata M Roem.; Azadirachta indica Adr. Juss.,; Melia azedarach L.; Chickrassia tabularis A. Juss.; Swietenia mahagoni L. Jacq.; Soymida febrifuga A. Juss; Cedrela odorata L.; Khaya grandifolia C.Dc.; Pseudocedrela kotschyi (Schweinf) Harms.; Entandrophragma spp.; Leguminosae (Fabaceae) [Bauhinia species; Delonix regia (Boj. ex Hook.) Raf.; Butea monosperma (Lam.) Taubert; Pterocarpus marsupium Roxb.; Sesbania grandiflora (L.) Pers.; Cassia fistula L.; Adenanthera pavonina L.; Astragalus spp.; Tamarindus indica L.; Pongamia pinnata (L.) Pierre; Parapiptadenia rigida (Benth.) Brena; Dicorynia paraensis Benth.; Caesalpinia. Spp.; Pithecellobium spp.; Leucaena spp.; Lysiloma acapulcense (Kunth) Benth.; Inga stipularis DC.; Prosopis spp.; Acacia spp.; Albizia; Dichrostachys cinerea (L.) Wight & Arn.; Afzelia africana Pers.; Brachystegia spiciformis Benth.; Julbernardia spp. Julbernardia globiflora (Benth.) Troupin; Isoberlinia scheffleri (Harms) Greenway; Burkea africana Hook; Cordyla africana Lour.; Virgilia oroboides (P. Bergius) T.M. Salter; Entada africana Guillemin & Perrottet; Erythrophleum africanum (Benth.) Harms; Piptadeniastrum africanum (Hook.f.) Brenan; Cercis siliquastrum L.; Parkia bicolor A. Chev.]; Rhizophoraceae [Rhizophora mangle L.]; Orchidaceae [Geodorum nutans (Presl) Anzes]; Celastraceae [Elaeodendron roxburghii W.; Cassine aethiopica Thunb.]; Moringaceae [Moringa oleifera Lam.]; Sapindaceae [Sapindus trifoliatus L.; Talisia olivaeformis (CH.B.&K.) Radlk.; Atalaya hemiglauca F. Muell. ex Benth.]; Annonaceae [Saccopetalum tomentosum Hk. F. et Th.]; Burseraceae [Garuga pinnata Roxb.]; Bombacaceae [Bombax ceiba Linn.; Adansonia spp.; Chorisia speciosa st. Hill.]; Arecaceae [Cocos nucifera L.; Borassus flabellifer L.]; Lythraceae [Lagerstroemia parviflora Roxb.]; Lecythidaceae [Careya arborea Roxb.]; Boraginaceae [Cordia myxa L.]; Malvaceae [Thespesia populnea (L.) Soland. ex Correa]; Cactaceae spp. [Opuntia spp.; Pereskia spp.]; Bromeliaceae [Puya chilensis Mol]; Guttiferae [Symphonia globulifera L. f.]; Sapotaceae [Bassia longifolia L.; Thevetia peruviana (Pers.) K. Schum.]; Pinaceae [Larix occidentalis Nutt.]; Capparidaceae [Crataeva adansonii DC.; Capparis nobilis F Muell.]; Araliaceae [Cussonia arborea Hochst. ex A. Rich; Schefflera volkensii (Engl.) Harms.; Polyscias sambucifolia (Sieber ex DC.)]; Vitaceae [Cissus populnea Guill. & Perr.]; Penaeaceae [Penaeae spp.]; Zamiaceae [Encephalartos spp.]; Cycadaceae [Cycas lane-poolei C.A. Gardner; Cicas revoluta Thunb.]; Stangeriaceae [Stangeria eriopus (Kunze) Nash]; Ebenaceae [Diospyros mespiliformis Hochst ex A. DC.]; Agavaceae [Phormium tenax JR & G. Forst.]; Elaeocarpaceae [Sloanea australis F. Muell.; Elaeocarpus reticulates]; Cunoniaceae [Ceratopetalum apetalum D. Don]; Gymnospermae [Araucaria spp.]; Ochnaceae [Lophira alata Banks ex Gaertn. f.]; Rhamnaceae [Ziziphus Juss.]; Euphorbiaceae [Aleurites moluccana (L.) Willd.]; Pittosporaceae [Pittosporum phillyreoides DC.; Actinidia spp.].*

### Preparation of hydrogels

The present invention further provides methods for the preparation of a hydrogel having the aforementioned properties. The order of steps, the amount of the ingredients, and the time of mixing are important process variables which will depend on the specific polymers, active agents, solvents and/or non-solvents, modifiers, additives and excipients used in the composition. These factors can be adjusted by those skilled in the art, while focusing on the objective of achieving a hydrogel product.

The present invention provides a method for the preparation of a bioadhesive hydrogel pharmaceutical composition comprising 10% to 50% w/w of at least one modified hydrophilic polysaccharide; 20% to 50% w/w of at least one non-solvent of the polysaccharide; 10% to 40% w/w of at least one solvent of said polysaccharide; and 10% to 40% w/w of at least one humectant; wherein the modified hydrophilic polysaccharide is selected from a partially depolymerized polysaccharide, borate ion cross-linked polysaccharide and an acid cross-linked polysaccharide, the method comprising the steps of:
a) exposing said polysaccharide to a modifying agent;
b) dispersing at least one polysaccharide in at least one non-solvent of said polysaccharide;
c) admixing said solvent with the at least one humectant;
d) combining said polysaccharide-non-solvent dispersion with said solvent-humectant mixture to generate a viscous mixture; and
e) dispensing said viscous mixture.

As one of ordinary skill in the art would appreciate, other sequences of steps may be possible. Therefore, the particular order of the steps set forth in the specification should not be construed as limitations. For example, the polysaccharide may be modified at any of the steps of the method. In certain embodiments the modification of the polysaccharide may precede or follow its dispersion in the non-solvent. In one embodiment the polysaccharide is modified by ozone-UV irradiation, following dispensing of the viscous mixture. In another embodiment the polysaccharide is depolymerized by gamma irradiation, sonication, mechanical pressure and heating before dispersing in the non-solvent.

### Type I Modification

Type I modifications include those, which yield a partially depolymerized polysaccharide having a molecular weight lower than the molecular weight of the corresponding native polysaccharide. A "lower molecular weight" refers to a polysaccharide having a molecular weight (MW) that is 10% to 99% of the MW of the corresponding native polysaccharide. In certain embodiments the MW of the partially depolymerized polysaccharide is 20% to 80% of the MW of the native polysaccharide. In specific embodiments the MW of the partially depolymerized polysaccharide is 25% to 75% of the MW of the native polysaccharide. The hydrogel of the present invention is produced by mixing a polysaccharide powder with a non-solvent for the polysaccharide together with a solvent phase, which preferably contains humectants such as glycerol to form a viscous feed. In a non-limiting example the molecular weight of karaya gum is about 9.5 × 10⁶ Dalton. The preferred MW of karaya gum is 0.1 × 10⁶ Dalton to 9 × 10⁶ Dalton.

The particle size of the polysaccharide powder has an effect on the final viscoelasticity and tack of the compositions. Particle sizes of 20-300 micron can be utilized. Preferred particle size is about 200 micron.

A convenient procedure is to combine the two phases at temperatures lower than -5°C to delay hydrogen bonding reaction. Preferred temperature is about -10°C.

The composition may further comprise a polyol. Without wishing to be bound to theory, the presence of polyol may enhance the molar concentration of the polysaccharide's free carboxylic acid to improve hydrogen bonding with any polar surface or ionic bonds with polycationic surfaces. Alcohol and polyols may also serve as a penetration enhancers in transdermal applications.

### Type II Modification

A polysaccharide is used in combination with the non-solvent as described in preparation Type I. Boric acid is added to water and heated to 60°C to full dissolution. Glycerol is than added to the solution followed by concentrated base, preferably a sodium hydroxide solution. The amount of water in the hydrogels is critical as it drastically alters the swelling capacity during coating and the viscoelasticity and tack of the final product. Accordingly, the amount of water content in the sodium hydroxide solution must be balanced with the total formulation. Use of a sodium hydroxide solution having a concentration of 30% to 70% is preferred. The water and polysaccharide-non-solvent phases are combined as described in preparation Type II.

The method of preparing a hydrogel further comprises the step of adding an acid followed by a base. Therefore, a method for preparing a hydrogel comprising at least one modified polysaccharide comprises the following steps:
a) dispersing at least one polysaccharide in at least one non-solvent of said polysaccharide;
b) admixing the solvent with the at least one humectant;
c) combining said polysaccharide-non-solvent dispersion with said solvent-humectant mixture to generate a viscous mixture;
d) exposing the viscous mixture to borate ions or a volatile acid;
e) dispensing said viscous mixture.

The particular order of the steps set forth in the specification should not be construed as limitations.

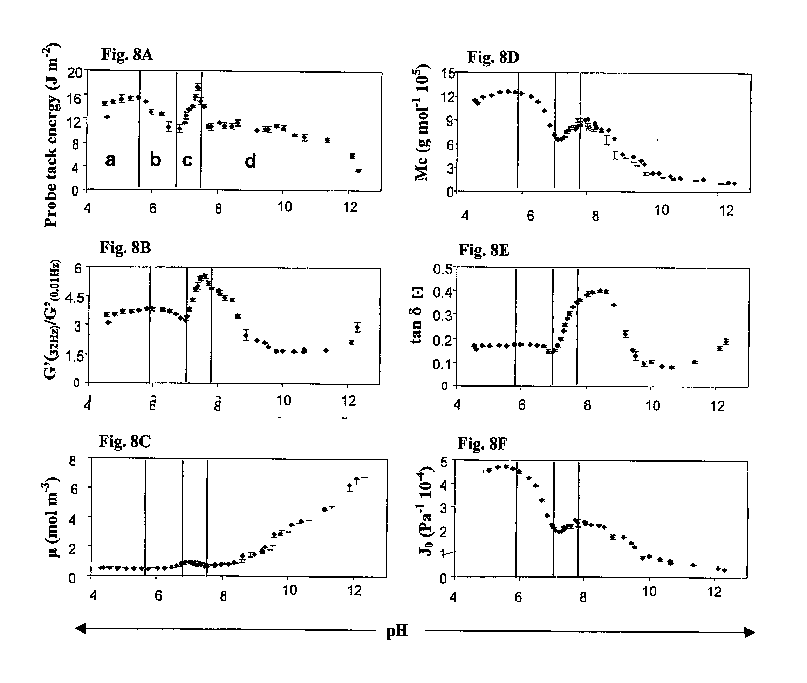

The hydrogels of the present method may be prepared under a wide range of pH, from pH 2 to pH 11. This wide range of pH permits the accommodation of a large variety of bioactive, cosmetic or therapeutic agents. For dermal applications, the hydrogels have a pH that is preferably within physiological range, i.e. pH 4 to about pH 8. For transdermal applications, the hydrogels may have a pH of pH 3 to pH 10 and still retain excellent tack (see figure 8A).

### Coating and Casting

The shape and method of shaping or casting the compositions of the present invention is not limiting and the compositions may be shaped by, for example, coating, casting or injectable molding.

The Type I or Type II mixture may be cast into a preshaped mold of any shape or size, depending on the application. Coating of the viscous mixture may be performed by methods known in the art. In certain embodiments the mixture is coated on a thin polymeric sheet e.g., polyethylene, polyester or polyamide preferably treated to prevent delamination during removal. After setting, the hydrogel may be backed on its exposed upper side with backing layer, such as silicone coated paper and cut to desired size and shape. Any quantity of the viscous dispersion may be applied to a backing film to form a continuous sheet of hydrogel 0.1 to 2 mm thickness by coating techniques or up to 10 mm or more by casting techniques. The viscosity of the dispersion and the preset gap of the coating machine dictate the thickness of the hydrogel applied to the backing. The backing provides strength and integrity to the adhesive patch and acts as a substrate for receiving and retaining a portion of the hydrogel.

### Backing Layer

The bioadhesive composition of the present invention is self-adhesive, i.e. capable of attaching to the site of application without the need to reinforce such attachment by means of the use of another adhesive applied over it or to a backing. Although in certain applications a backing layer is desirable.

The bioadhesive compositions of the present invention may optionally further comprise a backing layer, which conforms to the size and shape of an individual dosage unit or delivery system.

The backing layer, which may serve as the upper surface of the hydrogel, provides the hydrogel with flexibility and handling ability. The backing layer may be either occlusive or nonocclusive, depending on the application, i.e. whether it is desired that the skin become hydrated during drug delivery. In certain embodiments the composition further comprises a release liner, present on the composition side opposite to the backing layer.

The backing layer is typically extremely thin, and generally very flexible, and conformable to anatomical surfaces. As such, when the composition comprising a backing layer is applied to an anatomical surface, it conforms to the surface even when the surface is moved.

The backing layer is preferably made of a sheet or film of a preferably flexible elastomeric material. The material for the backing material may be selected so that it is substantially impermeable to the pharmaceutically active agents and to any other components of the composition, thus preventing loss of any components through the upper surface of the hydrogel. Suitable backing materials for the backing layer include, for example, synthetic and natural materials, non-woven fibrous webs, woven fibrous webs, knits, films polymers and other backing materials known in the art.

In some embodiments the hydrogel is treated with ozone and UV radiation (O₃/UV) prior to attachment of the backing.

### Adhesion measurements

The adhesiveness of the hydrogel sheet can be quantified by the probe-tack test method. This test method is detailed in the American Society for Testing Materials, Designation D-2979-01, under the jurisdiction of ASTM Committee D-14.50 on adhesives.

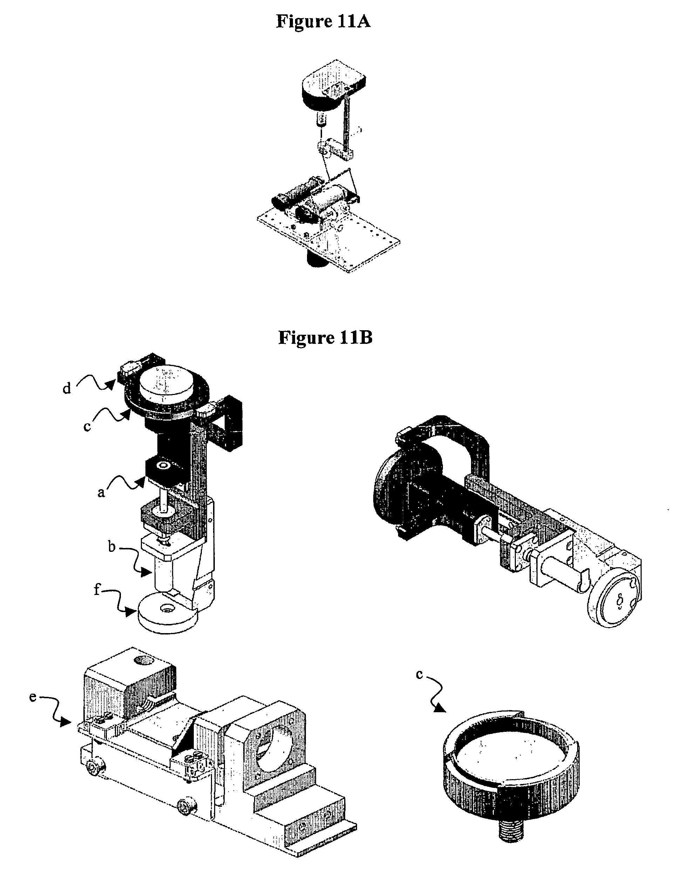

The polysaccharide-based hydrogels in this invention were tested using a novel probe tack tester device (Figure 11B). The novel apparatus is designed to test tacky hydrogels. Dwell times can be prefixed to the time period from first contact to debonding or alternatively, from the point of preset load to debonding. Bond formation in hydrogels can be in the order of 0.1 milliseconds (ms). It is more realistic to calculate dwell-time from the beginning of contact rather than from the achieved prefixed load. During compression to reach a specific load the time consumed may exceed the orders of milliseconds and this consumes a major portion of the realistic bond formation period. The present invention is directed to a device capable of detecting the contact level of the probe and hydrogel and to signal this level as the initial of dwell-time. Thus, the present invention further provides a device for detecting the tack of a soft PSA in general and of hydrogels in particular. Specifically, the device comprises a probe, a sensing device and means for detecting the energy during debonding under predetermined pressure. Specifically, the device tests probe tack in accordance to the American Society for Testing and Materials (ASTM) standards.

In one embodiment the device operates as follows: A probe, connected to the UTM load cell is lowered towards the hydrogel. The motion of the probe as well as the target load is determined by the UTM. Prior to lowering of the probe, the stage moves to a home positioning point, i.e., a point where the photoelectric sensing device recognizes the surface of the hydrogel and signals the controller. Hydrogels prepared by casting technique (using molds) are flat and homogeneous providing liable surface for accurate sensing. When the probe crosses the photoelectric beam a second signal is sent to the controller and the stage is traveled at predetermined velocity. The time period from the second signal to debonding is the dwell-time and is preset by the software from 1 ms to infinity. The time necessary for the probe to cross the beam (approximately 0.1 mm, is depended on the type of sensing device) is pre calculated and added to the total dwell-time.

Debonding occurs during the Z-motion of the stage in downward course. The force at debonding is recorded via the UTM. The UTM is interfaced with personal computer and computer program to perform data acquisition and conversion of the continuous voltage vs. time output into digitized force-time values. Special software is used to convert the force versus time to adhesion energy by integration of the force versus displacement. Additionally, the software analyses the following parameters: time periods from contact to debonding (or alternatively from loading to debonding) and time period of debonding. By using the latter time parameter the elongation (strain) can also be calculated.

A typical illustration curve demonstrating the operation modes of the test is presented as follows. The curve shows the time periods for compression to a predetermined load followed by sample relaxation and debonding. The dwell-time can be calculated from initial contact to initial debonding.

### Hydrogel Applications

The hydrogel formulations of this invention are intended to be used in a plurality of pharmaceutical and cosmetic applications including as adhesive patches, wound dressings, bioelectrodes, topical cosmetics, and as a drug reservoir in systems for passive transdermal drug delivery and electrotransport drug delivery. Cosmetic and pharmaceutical active agents can be included in the compositions of the present invention. A pharmaceutical or therapeutic agent is intended for topical treatment of human and animal diseases and that can be treated by topical application or transdermal delivery of an agent. Cosmetic agents are intended for beautifying or protecting the skin or for improving its appearance and or texture.

The composition of the present invention may further comprise one or more additives useful in the preparation or application of topically applied devices and compositions. For example, solvents, including alcohol, may be used to solubilize certain active agents. For pharmaceutically active agents having a low rate of permeation through the skin, it may be desirable to include a further permeation enhancer in the composition. Enhancers should be chosen to minimize the possibility of skin irritation, damage, and skin and systemic toxicity. Examples of suitable enhancers include, in a non-limiting manner, ethers such as diethylene glycol monoethyl ether (Transcutol®); surfactants such as sodium laurate, sodium lauryl sulfate (SLS), cetyltrimethylammonium bromide (CTAB), Poloxamer (231, 182, 184), Tween (20, 40, 60, 80) and lecithin; alcohols such as ethanol, propanol, octanol, benzyl alcohol, and the like; polyethylene glycol (PEG) and esters thereof; amides and other nitrogenous compounds such as benzalkonium chloride, urea, dimethylacetamide (DMA), dimethylformamide (DMF), 2-pyrrolidone, 1-methyl-2-pyrrolidone, ethanolamine, diethanolamine and triethanolamine; terpenes; alkanones; and organic acids. The permeation enhancers may in some instances provide more than one benefit or operate via more than one mode of action. For example, benzalkonium chloride may be used as a preservative.

The classification of agents used herein is made for the sake of convenience only and is not intended to limit any component to that particular application or applications listed.

### Therapeutic agents

A therapeutic agent or a combination of therapeutic agents may be incorporated in to the composition of the present invention for the delivery of the agent to a subject in need thereof. Therapeutic agents useful in connection with the present invention include any pharmaceutical compound or chemical that is capable of being administered to the skin or mucosal tissue or by passive transdermal or transmucosal delivery or by electrotransport. In general, this includes agents in all of the therapeutic areas including, but not limited to, anti-microbial agents including antibiotics, antifungal and antiviral agents; bacteriostatic agents; analgesics and analgesic combinations; anesthetic agents; anorexic agents; antiarthritic agents; antiasthmatic agents; anticonvulsants; antidiabetic agents; antiemetic and antidiarrheal agents; antihistamines; anti-inflammatory (steroidal and non-steroidal) and antipruritic agents; antimigraine preparations; antineoplastics; psychotherapeutics; antipyretics; antispasmodics; antiarrhythmics; antihypertensives; opioid antagonists; hormones; as well as pharmaceutically acceptable salts and esters thereof.

The present invention is also useful in conjunction with the topical or transdermal delivery of proteins, peptides and fragments thereof. The amount of therapeutic agent that constitutes a therapeutically effective amount can be readily determined by those skilled in the art with due consideration of the particular agent, the particular carrier, and the desired therapeutic effect.

### Cosmetic Agents

The hydrogels of the present invention are useful for the topical delivery of cosmetic agents and cosmeceuticals. General examples include anti-acne and anti-sebum agents, anti-oxidants, anti-aging, anti-scar and scar-, wrinkle- and pigment-reducing agents and moisturizers. The term "cosmeceutical" refers to an agent that provides cosmetic benefits such as vitamins, phytochemicals, enzymes, antioxidants, and essential oils.

Non-limiting examples anti-acne agents include agents that control or treat comedogenesis, sebum production, bacteria, and inflammation and embrace antibiotics, retinoids, benzoyl peroxide, vitamins and vitamin derivatives, sulfur containing agents, salicylic acid, azelaic acid and alpha hydroxy acid.

Examples of anti-oxidants and anti-wrinkle agents include alpha hydroxy acid, vitamins and vitamin derivatives, nicotinamide and derivatives thereof, Coenzyme Q10, polyphenols and catechins, phospholipids, amino acids and peptides, proteins and glycosaminoglycans.

The following examples will further describe the invention, and are used for the purposes of illustration only, and should not be considered as limiting the invention being disclosed.

### EXAMPLES

Percent (%) as used in the following examples, refers to percentage of weight of the ingredients per total weight of the formulation; PS refers to polysaccharide; MW refers to molecular weight.

### Example 1: Probe Tack Energy and Yield Stress of Unmodified (Native) Polysaccharides

Hydrogels comprising varying amounts of water and native *Sterculia urens* polysaccharide were prepared and tested for probe tack energy and yield stress.

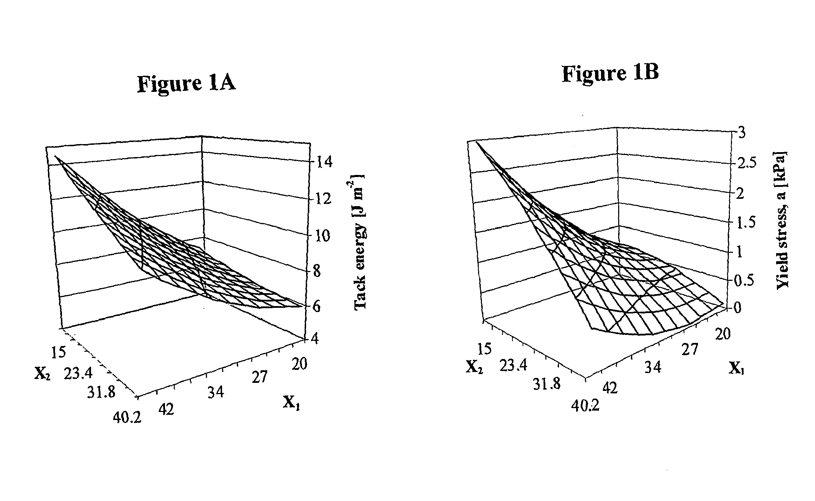

Figure 1 shows 3D graphs representing response surface methodology for optimization of the water and *Sterculia urens* polysaccharide ratio. Parameters used: probe tack energy and yield stress (rigidity) are calculated from a 3-parameter power-law model. Probe: Aluminum, diameter:22 mm; Bonding: 1N; 70 mm/min; Dwell-time: 1s; Debonding: 600 mm/min.

In general, an increase in the water content to a maximum volume resulted in an increase of swelling around acetyl groups, leading to the highest viscosity during the coating process. A combination of maximum water content and minimum polysaccharide content results in reduced rigidity and decreased tack. Maximal tack energy is attained with a maximal polysaccharide concentration and minimal amount of water. The rigidity of such a combination is still low relative to other high crosslinked hydrogel systems.

### Example 2: Hydrogel prepared with Type I Modified Polysaccharide

| **Component** | **Specific Component** | **%(w/w)** |
|---|---|---|
| PS | Sterculia urens, MW: 10⁶ Dalton | 26 |
| Non-solvent | propylene glycol | 30 |
| Solvent | deionized water | 20 |
| Humectant | glycerol | 24 |

The polysaccharide powder (Karaya, MW 9.5 × 10⁶) was depolymerized by gamma irradiation in a continuous cobalt 60 irradiator at doses of 1-70 kGy. The irradiation process was performed according to "Sterilization of health care products-requirements for validation and routing control-radiation sterilization" (ANSI/AAMI/ISC 11137:199595).

Other treatments (sonication, mechanical pressure, heating) were tested following dispersion of native powder in water.

The final product was manufactured by first mixing the PS (200 micron particle size, MW 0.1 - 9.5 × 10⁶) with the non-solvent for the polysaccharide at room temperature (25°C) until full dispersion was attained (Phase I). In parallel, the solvent was stirred with the humectant (Phase II). Both phases were stored at -18°C for 1 h, and mixed at 4°C to generate a viscous phase combination. The viscous feed was coated on a Corona treated polymeric sheet (for example polyamide) using a standard coater to form a 0.5 mm sheet and allowed to set for 1 h. After setting, the hydrogel was backed on its exposed upper side with a peelable release hydrophobic film, such as silicone coated paper and cut to the desired size and shape.

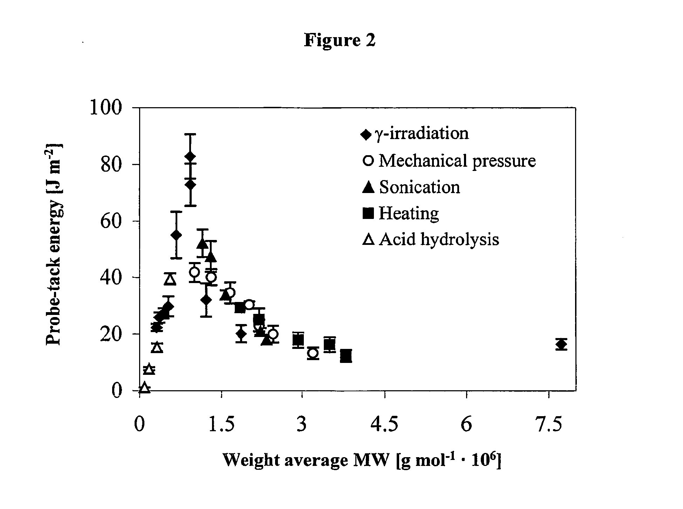

Figure 2 demonstrates a master curve for probe tack energy vs. molecular weight average of *S. urens* based hydrogels treated with a number of environmental stimuli including gamma irradiation, mechanical pressure, sonication, heating and acid hydrolysis. The average molecular weight of the various treated polysaccharides was determined by Multi Angle Laser Light Scattering (MALLS) and Size Exclusion Chromatography (SEC) and calculated via Debye or Berry models. The curve clearly shows that good correlation is obtained for probe tack energy vs. molar mass regardless of depolymerization routine. For example, gamma irradiation of the polysaccharide provides a wide range of molecular weights, from about 0.1 × 10⁶ to about 9 × 10⁶ Dalton, when the starting material is a 9.5 × 10⁶ Dalton native polysaccharide.

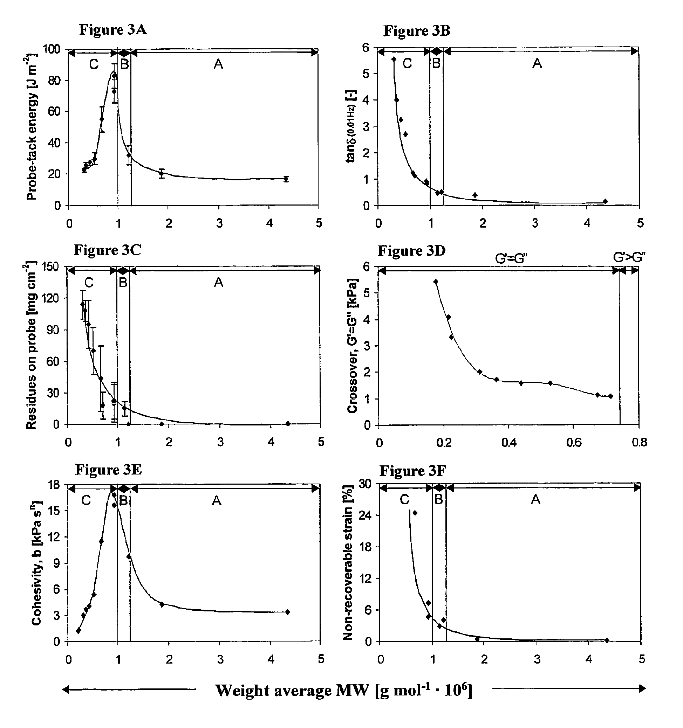

Figure 3 represents *S. urens* polysaccharide-based hydrogels depolymerized by gamma irradiation. The curves shows the probe tack energy at debonding along with the tangent δ, residues left on the probe, crossover point, , the cohesion indication calculated from a 3-parameter power law model and the non-recoverable strain after relaxation (calculated from creep-recovery curves). Three regions are demonstrated in each graph: regions A, B and C. In region C, at PS molecular weights of 300,000-500,000 Daltons, probe tack of the hydrogels is low (figure 3A) comparable to low cohesion (figure 3E) and high tangent δ (figure 3B). The same results are obtained in region A where molecular weights are 2-4 × 10⁶ Daltons, except the tangent δ is lower to 1 (figure 3B). Maximal tack energy is correlated with maximal cohesion and tangent δ tends to zero. In this maximum region, the proportion of elastic and viscous modulus is equal. The results demonstrate that restricted or partial depolymerization is accounted for a balanced adhesion to cohesion ratio to enables maximum in tack energy. Region C also shows a crossover point for every molecular weight indicative of a transition from sol to gel. Such points cannot be found for hydrogels of region A. In region C the failure is cohesive while in region A it is adhesive. Region B is a transition zone characterized by fibrils formation. For practical applications it is preferred to utilize hydrogels exhibiting properties of region A, i.e. those exhibiting adhesive failure, the failure to leave a residue upon debonding, rather than region B or C.

### Example 3: Hydrogel prepared with Type I Modified Polysaccharide

| **Component** | **Specific Component** | **%(w/w)** |
|---|---|---|
| PS | *S. urens,* MW: 7.7 × 10⁶ Dalton | 13 |
| PS | *S. urens,* MW: 3 × 10⁵ Dalton | 13 |
| Non-solvent | propylene glycol | 30 |
| Solvent | deionized water | 20 |
| Humectant | glycerol | 24 |

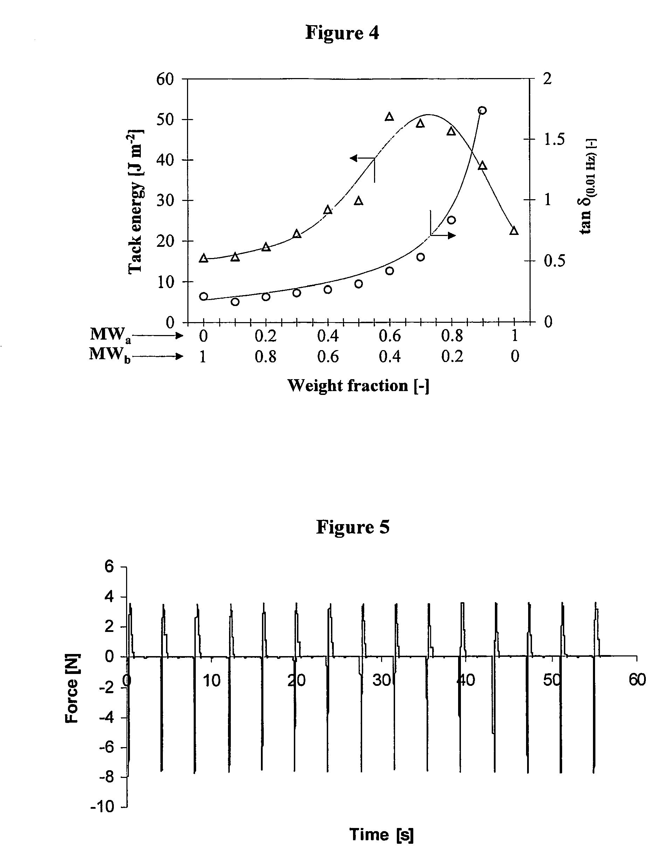

The procedure set forth in Example 1 is used with appropriate substitution of quantities to prepare this formulation. To improve adhesion performance, combinations of low and high molecular weights are employed. Figure 4 shows that by increasing the proportion of a low molecular weight fraction relative to a high molecular weight fraction it is possible to increase tack energy to the same values as found for the maximum (Figure 3) for hydrogels made of a single fraction with intermediary molar mass (~1 × 10⁶ Dalton) while maintaining adhesive failure. Probe tack energy and tangent δ for hydrogels composed of low, Fraction A (3 × 10⁵ Dalton) and high, Fraction B (4.4 × 10⁶ Dalton) molecular weight blends of *S. urens* polysaccharide. (Aluminum probe, diameter: 22 mm; Bonding: 0.5 N; 70 mm/min; Dwell-time: 1s; Debonding: 600 mm/min). The extent of elastic and viscous components determined the cohesive properties of the hydrogels as shown from tangent δ. Tangent δ equals ~1.5 provides maximum in tack energy. Using combinations of various molecular weights of one polysaccharide or polysaccharide blends improves adhesion over a wide range of applications.

### Example 4: Repositioning capability of Type I hydrogels.

Figure 5 demonstrates the repositioning ability of the modified type I polysaccharide-based hydrogels. The curve represents the force vs. time curve for hydrogel composed of low and high molecular weight fraction combinations (3 × 10⁵ and 4.4 × 10⁶ Daltons, respectively) in a ratio of about 1:1. The graph shows that the hydrogels do not lose their tack properties even after 15 cycles under a bonding pressure of 5 N, using an aluminum probe. No residues were found on the probe after test ending. Probe: Aluminum, diameter:22 mm; Bonding: 5N; 500 mm/min; Dwell-time: <1 s; Debonding: 600 mm/min.

### Example 5: Hydrogel prepared with Type I Modified Polysaccharide: UV-Ozone Treatment

| **Component** | **Specific Component** | **%(w/w)** |
|---|---|---|
| PS | *S. urens,* MW: 4 × 10⁶ Dalton | 24 |
| Non-solvent | propylene glycol | 32 |
| Solvent | deionized water | 20 |
| Humectant | Glycerol | 24 |

The procedure set forth in Example 1 is used with appropriate substitution of quantities to prepare this formulation with the addition of exposing the hydrogel sheets to a UV-Ozone chamber for 2 minutes before backing with release silicone type paper. The hydrogel is molded (or made as a sheet) as a single layer. Without wishing to be bound to theory, the high irradiation energy due to the ozone in the UV chamber modifies the surface of the hydrogel (reduces its MW) and a gradient of structures is formed, i.e., the upper layers being more soft and tacky where the internal layer or the bottom layer is non affected. In parallel, the ozone increases the quantity of hydroxyls and the hydrogels become more hydrophilic. Thus, the overall gel remains cohesive and with high integrity without the need of a backing support, yet very tacky and hydrophilic on the surface.

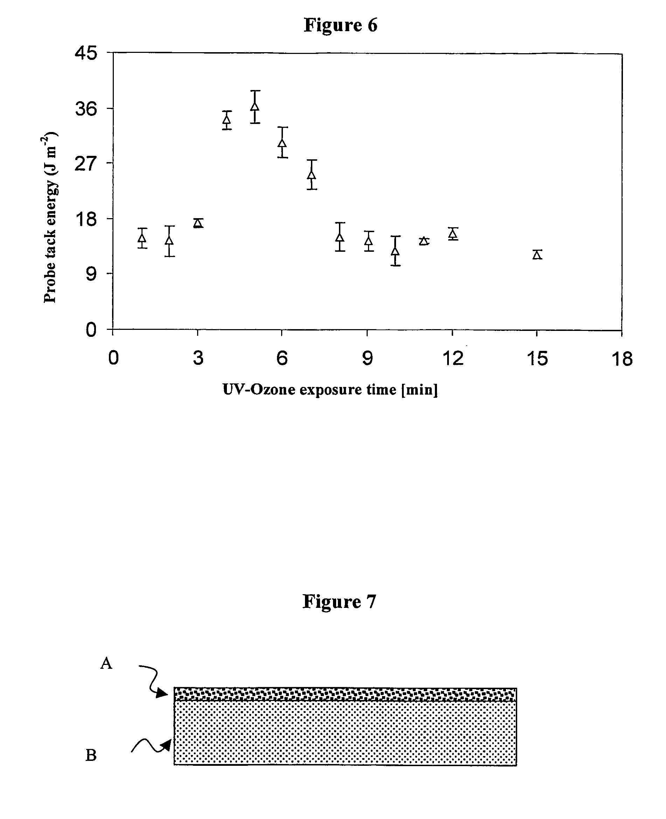

Figure 6 represents the probe tack energy for *S. urens* based hydrogels exposed to varying times in a UV chamber (1-15 min), tested using an aluminum probe. (Aluminum probe, diameter: 22 mm; Bonding: 0.5 N; 70 mm/min; Dwell-time: 1s; Debonding: 600 mm/min). Figure 6 shows that UV-ozone treatment can significantly increase tack energy with increasing exposure time. This is due to a simultaneous depolymerization process and increased hydrophilic character, as demonstrated by increase of hydroxyl groups. Maximum in probe tack occurs after 5 minutes exposure. A further increase in exposure time decreases the probe tack energy due to excess depolymerization, as shown earlier for irradiated polysaccharides of *S. urens.* Hydrogels treated by O₃-UV show an adhesive type failure up to exposure time of 6 minutes. Cohesive type failure is evident from 7-15 minutes. Without wishing to be bound to theory, the UV radiation converts the free flow of oxygen in the chamber to ozone and consequently increases the hydrophilic character of the polysaccharide simultaneously to depolymerization. This type of polysaccharide modification occurs only in the vicinity of the exposed surfaces, thus, a product with high integrity having tacky surface is obtained as illustrated in **Figure 7****.** It was shown that the molar concentration of the hydroxyl groups for hydrogels exposed to UV-Ozone treatment after 1 min was 18 mmol/g comparing a value of 6 in the native form. The content of hydroxyl groups in the polysaccharide was calculated from surface fractions of the hydrogels after drying in ethanol followed by evaporation. A calibration curve for glucose using FTIR was employed.

### Example 6: Hydrogel prepared with Type II Modified Polysaccharide

| **Component** | **Specific Component** | **%(w/w)** |
|---|---|---|
| PS | *S. urens,* MW: 4 × 10⁶ Dalton | 24 |
| Non-solvent | propylene glycol | 30 |
| Solvent | deionized water | 15 |
| Humectant | Glycerol | 22 |
| Acid | Boric acid | 3 |
| Base | Sodium hydroxide (70%) | 6 |

The powdered polysaccharide (karaya gum, MW 9.5 × 10⁶) was dispersed in the non-solvent as described in Examples 1 and 2 for Modification type I. Alternatively, the polysaccharide gum exudate of *Acacia* (e.g. gum Arabic) is used. Boric acid was added to water and heated to 60°C to full dissolution. Glycerol was then added to the solution followed by concentrated sodium hydroxide solution (70%). The molding procedure set forth in Example 1 was used.

To initiate crosslinking, hydroxide ions were added to shift the boric acid equilibrium in favor of the borate ion, as follows:

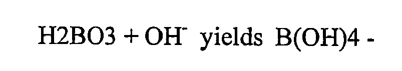

The pKa for the equilibrium reaction of an aqueous boric acid solution is 9.14. Without being bound to theory, the cis-diol groups on the sugar monomer units complex with the borate ions. Two cis-diol pairs on different PS molecules can thus be connected by a borate ion to form an interchain crosslink. Increasing the pH in the hydrogel to no more than about pH 11 increases the concentration of borate ions added to the system and consequently, tetrafunctional crosslinking increases. Increasing the crosslinking increases the cohesiveness.

Figure 8 depicts the properties of a hydrogel prepared using the type II modification. *S. urens* polysaccharide-based hydrogels were tested with an aluminum probe. 8A: probe tack energy: (probe: aluminum, diameter: 22 mm; bonding: 1N, 70 mm/min; Dwell-time: 1s; Debonding: 600 mm/min); 8B: Ratio of storage modulus at 32 and 0.01 Hz (high and low oscillation frequency, respectively); 8C: cross-linking density; 8D: average molecular weight between cross-links; 8E: tangent δ; 8F: creep compliance.

The novel hydrogels of this type are made over a wide range of pH and viscoelastic properties. Increasing the NaOH concentration (pH 4-5.5 region **a**) increases the debonding energy to a maximum point (curve 8A). The concentration of borate ions is too low to promote functional cross-linking, thus the cross-linking density, µ (curve 8C) is not altered although averaged molecular weight between hydrogen bonding association points increases (curve 8D). Without wishing to be bound to theory, this change in tack is presumably due to a combined effect of the presence of salts, NaOH and boric acid, which leads to moderate breaking of the hydrogen bonding and expansion of the three-dimensional network. An expanded structure leads to increased molecular flexibility as seen from the increase in creep compliance, J₀ (Curve 8F).

Without wishing to be bound to theory, a slight increase of the crosslinking density (region **b**) and decrease in molecular weight between crosslinks in parallel to decreased wetting (decreased J₀; 8F) is a result of an increase in borate ion concentration to promote links via hydroxyl groups of the polysaccharide. A further increase in pH (region **c**) leads to a tack increase to the maximum point in region **d** (8A). Increasing tack in region **c** is due to combined elasticity and wettability providing good tack and increase in dissipation energy. The ratio of the storage modulus at high oscillation frequency (32Hz, equivalent to debonding) to low frequency (0.01 Hz, equivalent to bonding) demonstrates this phenomenon (Curve 8B). An increase of the creep compliance and tangent δ (Curve 8E) in region **c** proves that the viscous elements are increasing simultaneously to elasticity. At pH 7 the formation of carboxylate ions increases (data not shown) and this also leads to improved hydrogen, coordinate or ionic bonding with the probe. The ability of the hydrogels to increase the interfacial bonding enables the manifestation of the elastic components during debonding. In regions **c** and **d**, formation of the deacetylation product, sodium acetate, significantly contributes to molecular expansion. A further increase in pH, over 7.5, increases the cross-linking density, which dominates the other parameters and tack tends to decrease. This phenomenon can be seen in **d** and is supported by all presented rheological parameters. Increasing the pH from 4.5 to over 12 does not depolymerize the polysaccharide, as was verified from SEC and MALLS testing.

The probe tack energy curve (8A) clearly demonstrates that hydrogels having pH of up to at least pH 10 can be produced in by method and which have similar adhesion and rheological properties as gels at pH 4.5. Hydrogels with varying pH can be produced for specific topical applications including transdermal delivery for optimal accommodation and compatibility of drugs and minimization of skin irritation.

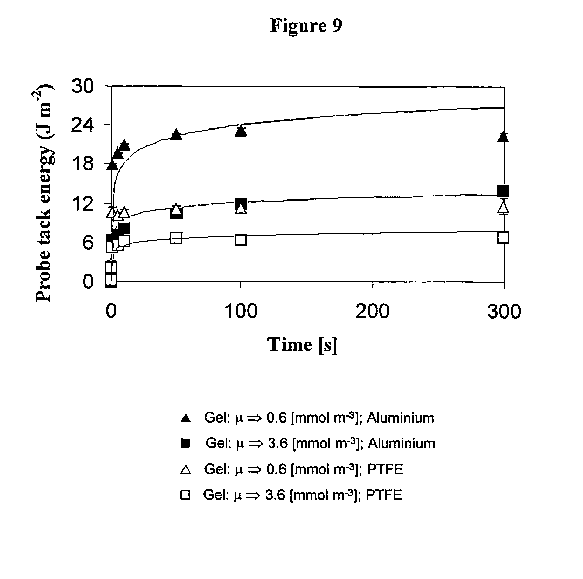

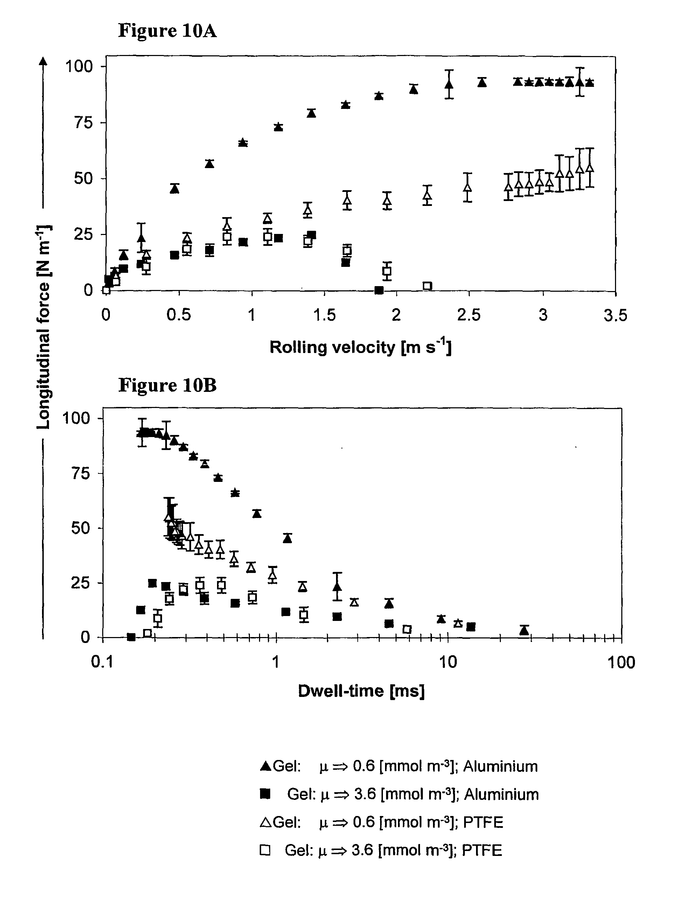

Figures 9 and 10 demonstrate that the type II hydrogels are capable of completing bond formation during 100 seconds dwell-time (Figure 9), while bond formation is in the order of 0.1-0.5 milliseconds (Figure 10), time periods applicable to PSA applications. Figure 9 represents the bond tack energy vs. dwell time employing the novel probe tack tester disclosed in the present invention (Aluminum and PTFE probes, 22mm diameter; Bonding: 70 mm/min; 1N; Debonding: 600 mm/min). Figures 10A and 10B represent the longitudinal force vs. rolling velocity and dwell-time, respectively, employing a rolling tack testing device by utilizing a modified Hertz equation based on contact mechanics. The points where debonding occurs before bonding is characterized by an extremum, which relates to the critical dwell-time for bond formation (Ben-Zion and Nussinovitch, 2002a,b, 2003; Nussinovitch, 2000).

### Example 7: Hydrogel prepared with Type II Modified Polysaccharide

A hydrogel having pH 7 can be obtained according the following example:

| **Component** | **Specific Component** | **%(w/w)** |
|---|---|---|
| PS | *Bauhinia variegata* (orchid tree) | 24 |
| Non-solvent | propylene glycol | 32 |
| Acid and solvent | 75 parts solvent (deionized water) + 25 parts HCl 37% | 20 |
| Humectant | glycerol | 24 |

Powdered *Bauhinia variegata* polysaccharide (commercially available from Krystal colloids, India, Brand KCH) is used in combination with the non-solvent as described in the aforementioned examples. Hydrochloric acid is added to the water phase followed by glycerol. The procedure set forth in Example 1 is used with appropriate substitution of quantities to prepare this formulation.

### Example 8: Probe-tack device

Figure 11A shows the rolling tack apparatus described previously (Ben-Zion and Nussinovitch, 2002a). Figure 11B shows the probe-tack device of the present invention. The apparatus comprises a high-performance DC-motorized linear positioning stage in the Z configuration (**a**) and enables fine mechanical resolution for precision micron stepping. The DC-micrometer (**b**) is coupled to an encoder connected to a motion controller (MCDC2805 Faulhaber, Schonaich, Germany) and interfaced to a host personal computer via an RS-232 port. Programmable software (e.g., Faulhaber Motion Manager) can be used for output motor configuration, online communication, sequencing velocity and timing profiles modes via ASCII terminal. The stage is equipped with a modular sample holder (**c**) specially designed for molded hydrogels. A pair of micro photoelectric thru-beam side type sensors (**d**) is mounted on both sides of the top sample holder. The distance of the paired sensors can be altered to increase the sensitivity of the beam for use in semi-transparent materials. The sensing device preferably contains 2-color indicators and a projected lead for alignment. The traveling range of the stage is predetermined via pairs of micro switches (**e**) and by the actuator length. For deformable hydrogels a traveling range of 20-100 mm is preferable. The assembly is mounted (**f**) on the floor of any Universal Testing Machine (UTM) e.g., Instron, in uniaxial position to the center of the load cell.

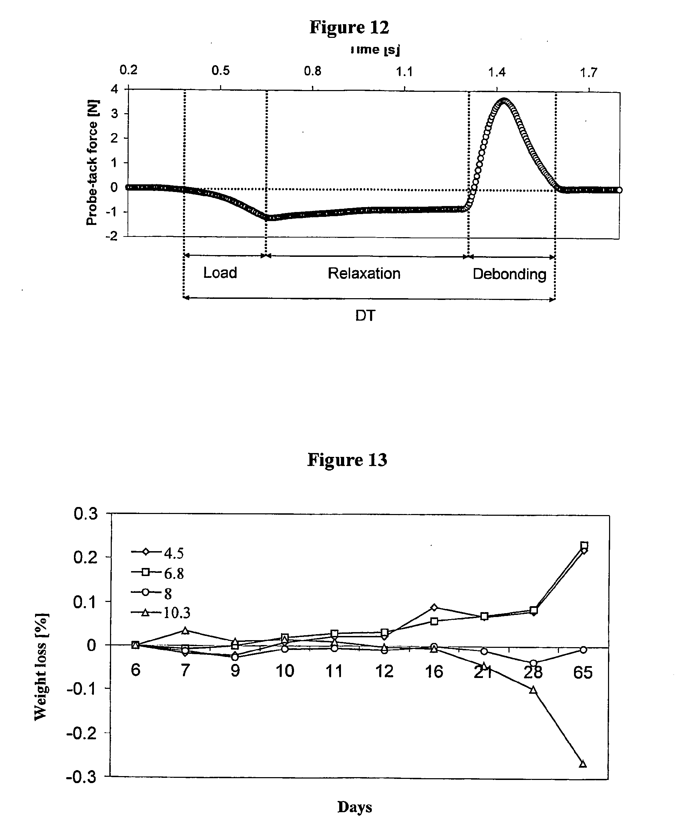

Figure 12 shows a typical curve demonstrating the operation modes of the test sample. The curve shows the time periods for compression to a predetermined load followed by relaxation and debonding. The dwell time can be calculated from initial contact to initial debonding.

### Example 9: Shelf Life of the hydrogels

Shelf life of the compositions of the present invention was determined by placing the material in a sealed aluminum packaging for various lengths of time. The weight of the hydrogel, adhesive properties and level of contamination were determined before and after the test period. The hydrogels retained their excellent properties even after 5 years in storage. The packaging material and methods of storage are not intended to be limiting.

Figure 13 is a graph that demonstrates the stability of the hydrogels to moisture loss. Type II hydrogels with various pH were kept in non-sealed containers for 65 days. Maximum weight loss for gels with pH of 4.5 and 6.8 after 65 days did exceed 0.2%. Gels with higher pH absorbed air humidity and increased their weight by 0.3%. Sealed storage conditions minimize the loss to a negligible value, (data not shown).

### REFERENCES

Benedek I, Heymans LJ. Pressure-Sensitive Adhesive Technology. NY: Marcel Dekker, 1997.
Ben-Zion O, Nussinovitch A. 2002a. Testing the Rolling Tack of Pressure-Sensitive Adhesive Materials. Part I: Novel Method and Apparatus. J. Adhesion Sci. Technol., 16(3):227-237.
Ben-Zion O, Nussinovitch A. 2002b. Testing the Rolling Tack of Pressure-Sensitive Adhesive Materials. Part II: Effect of adherend surface roughness. J. Adhesion Sci. Technol., 16(5):597-617.
Ben-Zion O, Nussinovitch A. 2003. Determination of green-bond strength in tacky Poly(vinyl alcohol) hydrogels. J. Appl. Polym. Sci., 87(13):2130-2135.
Bergman B, Lowhagen GB, Mobacken H. 1982. Irritant skin reaction to urostomal adhesives. Urol. Research 10:153-5.
Chen JL, and Cyr GN. 1970. Compositions producing adhesion through hydration. in: Manly RS, ed. Adhesion in Biological Systems. NY: Academic Press,; pg 163-81.
Goulding TM., 1994. Pressure-sensitive adhesives. In: Pizzi A, ed. Handbook of Adhesive Technology. N.Y: Marcel Dekker Inc., pgs 549-64.
Hammond FH. 1989. Tack. In: Satas D, ed. Handbook of Pressure-Sensitive Adhesives Technology. NY: Van Nostrand Reinhold, pgs 32-9.
Nussinovitch A, Ben-Zion O. Novel apparatus for testing the rolling tack of pressure-sensitive adhesive hydrogels. Provisional application filed 01/9/2000.
Tan HS, Pfister WR. 1999. Pressure-sensitive adhesives for transdermal drug delivery systems. Pharmaceutical Science & Technology Today, 2:60-9.

## Claims

1. A bioadhesive hydrogel composition comprising:
a) 10% to 50% w/w of at least one modified hydrophilic polysaccharide;
b) 20% to 50% w/w of at least one non-solvent of the polysaccharide;
c) 10% to 40% w/w of at least one solvent of said polysaccharide; and
d) 10% to 40% w/w of at least one humectant; wherein the modified hydrophilic polysaccharide is selected from a partially depolymerized polysaccharide, a borate ion cross-linked polysaccharide, and an acid cross-linked polysaccharide.

2. The composition according to claim 1, further comprising an active agent selected from a therapeutic agent and a cosmetic agent.

3. The composition according to claim 1 wherein the modified polysaccharide is a partially depolymerized polysaccharide.

4. The composition according to claim 3,
wherein the polysaccharide is partially depolymerized using a method selected from gamma irradiation, UV radiation, ozone exposure, sonication, mechanical pressure, heating and acid hydrolysis.

5. The composition according to claim 4 wherein the polysaccharide is partially depolymerized by gamma irradiation, preferably wherein the polysaccharide is partially depolymerized by ozone and UV radiation; or
wherein the polysaccharide is a polysaccharide exudate selected from the Sterculiaceae family and Leguminosea (Fabaceae family of herbs, shrubs and trees, preferably wherein the polysaccharide is selected from the group consisting of gum karaya and gum Arabic, more preferably wherein the polysaccharide is gum karaya; or
wherein the partially depolymerized polysaccharide has an average molecular weight of 0.1 x 10⁶ to 9 x 10⁶ Daltons; or
wherein the partially depolymerized polysaccharide has an average molecular weight of 0.2 x 10⁶ to 8 x 10⁶ Daltons.

6. The composition according to claim 3, comprising a high molecular weight fraction and a low molecular weight fraction of at least one partially depolymerized polysaccharide.

7. The composition according to claim 6,
wherein the high molecular weight fraction has a molecular weight of 2 x 10⁶ to 9 x 10⁶ Daltons and the low molecular weight fraction has a molecular weight of 0.1 x 10⁶ to 2 x 10⁶ Daltons; or
wherein the high molecular weight fraction, and the low molecular weight fraction comprise partially depolymerized karaya gum; or
wherein the high molecular weight fraction comprises partially depolymerized karaya gum and the low molecular weight fraction comprises Bauhinia variegata exudate.

8. The composition according to claim 1, wherein the modified polysaccharide is a borate ion crosslinked polysaccharide.

9. The composition according to claim 8,
wherein the borate ion source is selected from the group consisting of boric acid and sodium tetraborate decahydrate; or
wherein borate ion source is boric acid together with a base.

10. The composition according to claim 1 wherein the modified polysaccharide is a volatile acid crosslinked polysaccharide.

11. The composition according to claim, 10 wherein the volatile acid is hydrochloric acid.

12. The composition according to any one of claims 8 and 10, wherein the composition has a pH in the range from pH 2 to pH 11.

13. The composition according to any one of claims 8 and 11 wherein the hydrophilic polysaccharide is a polysaccharide exudate that derives from the group of herbs, shrubs and trees selected from the Sterculiaceae, Cochlospermaceae, Rutaceae, Proteaceae, Combretaceae, Rosaceae, Anacardiaceae, Meliaceae, Leguminosae, Rhizophoraceae, Celastraceae, Moringaceae, Sapindaceae, Annonaceae, Burseraceae, Bombacaceae, Arecaceae, Lythraceae, Lecythidaceae, Boraginaceae, Malvaceae, Cactaceae, Bromeliaceae, Guttiferae Sapotaceae, Pinaceae, Capparidaceae, Araliaceae, Vitaceae, Penaeaceae, Zamiaceae, Cycadaceae, Stangeriaceae, Ebenaceae, Agavaceae, Elaeocarpaceae, Cunoniaceae, Gymnospermeae, Ochnaceae, Rhamnaceae, Euphorbiaceae and Pittosporaceae families.

14. The composition according to claim 13 wherein the polysaccharide exudate is selected from Sterculia urens and Bauhinia variegata species.

15. The composition according to claim 1,
wherein the non-solvent is selected from propylene glycol, dipropylene glycol, polyethylene glycol, butylene glycol, hexylene glycol, poly oxy ethylene, polypropylene glycol and ethylene glycol, preferably wherein the non-solvent is propylene glycol; or
wherein the humectant is selected from glycerol, sorbitol and maltitol.

16. A method of preparing an adhesive hydrogel pharmaceutical composition according to claim 1, the method comprising the steps of:
a) exposing the polysaccharide to a modifying agent;
b) dispersing said the at least one polysaccharide in at least one non-solvent of said polysaccharide;
c) admixing said solvent with the at least one humectant;
d) combining said polysaccharide-non-solvent dispersion with said solvent- humectant mixture to generate a viscous mixture; and
e) dispensing said viscous mixture.

17. A bioadhesive hydrogel composition for use in a topical, systemic or transdermal administration of a pharmaceutically active agent comprising:
a) 10% to 50% w/w of at least one modified hydrophilic polysaccharide;
b) 20% to 50% w/w of at least one non-solvent of the polysaccharide;
c) 10% to 40% w/w of at least one solvent of said polysaccharide; and
d) 10% to 40% w/w of at least one humectant; wherein the modified hydrophilic polysaccharide is selected from a partially depolymerized polysaccharide, a borate ion cross-linked polysaccharide, and an acid cross-linked polysaccharide.

18. Bioadhesive hydrogel composition according to claim 1 for the topical, systemic or transdermal administration of a pharmaceutically active agent.

19. Use of a bioadhesive hydrogel composition according to claim 1 for the topical, administration of a cosmetic agent.

## Patentansprüche

1. Biologisch adhäsive Hydrogelzusammensetzung, umfassend:
a) 10 bis 50-Gew.-% mindestens eines modifizierten hydrophilen Polysaccharids,
b) 20 bis 50-Gew.-% mindestens eines Nicht-Lösungsmittels des Polysaccharids;
c) 10 bis 40-Gew.-% mindestens eines Lösungsmittels des Polysaccharids, und
d) 10 bis 40-Gew.-% mindestens eines Feuchthaltemittels, worin das modifizierte hydrophile Polysaccharid ausgewählt ist unter einem teilweise depolymerisierten Polysaccharid, einem mit Borat-Ionen vernetzten Polysaccharid und einem mit Säure vernetzten Polysaccharid.

2. Zusammensetzung nach Anspruch 1, die weiter ein aktives Mittel ausgewählt unter einem therapeutischen Mittel und einem kosmetischen Mittel umfasst.

3. Zusammensetzung nach Anspruch 1, worin das modifizierte Polysaccharid ein teilweise depolymerisiertes Polysaccharid ist.

4. Zusammensetzung nach Anspruch 3, worin das Polysaccharid unter Verwendung eines Verfahrens ausgewählt unter Gammabestrahlung, UV-Bestrahlung, Ozon-Exposition, Ultraschallbehandlung, mechanischem Druck, Erwärmung und saurer Hydrolyse teilweise depolymerisiert wird.

5. Zusammensetzung nach Anspruch 4, worin das Polysaccharid durch Gammabestrahlung teilweise depolymerisiert wird, vorzugsweise worin das Polysaccharid durch Ozon und UV-Bestrahlung teilweise depolymerisiert wird; oder
worin das Polysaccharid ein Polysaccharidexsudat ist, ausgewählt unter der Familie der Sterculiaceae und Legominosen (Fabaceae-Familie von Gewürzen, Sträuchern und Bäumen, vorzugsweise worin die Polysaccharide ausgewählt sind aus der Gruppe bestehend aus Karayagummi und Arabikumgummi, noch bevorzugter worin das Polysaccharid Karayagummi ist; oder
worin das teilweise depolymerisierte Polysaccharid ein durchschnittliches Molekulargewicht von 0,1x 10⁶ bis 9x 10⁶ Dalton aufweist, oder
worin das teilweise depolymerisierte Polysaccharid ein durchschnittliches Molekulargewicht von 0,2x 10⁶ bis 8x 10⁶ Dalton aufweist.

6. Zusammensetzung nach Anspruch 3, die eine Fraktion mit hohem Molekulargewicht und eine Fraktion mit niedrigem Molekulargewicht von mindestens einem teilweise depolymerisierten Polysaccharid umfasst.

7. Zusammensetzung nach Anspruch 6,
worin die Fraktion mit hohem Molekulargewicht ein Molekulargewicht von 2x 10⁶ bis 9x 10⁶ Dalton aufweist und die Fraktion mit dem niedrigen Molekulargewicht ein Molekulargewicht von 0,1x 10⁶ bis 2x 10⁶ Dalton aufweist; oder
worin die Fraktion mit dem hohen Molekulargewicht und die Fraktion mit dem niedrigen Molekulargewicht teilweise depolymerisierten Karayagummi umfassen; oder
worin die Fraktion mit hohem Molekulargewicht teilweise depolymerisierten Karayagummi und die Fraktion mit niedrigem Molekulargewicht *Bauhinia variegata* Exsudat umfasst.

8. Zusammensetzung nach Anspruch 1, worin das modifizierte Polysaccharid ein mit Borationen vernetztes Polysaccharid ist.

9. Zusammensetzung nach Anspruch 8,
worin die Borationenquelle ausgewählt ist aus der Gruppe bestehend aus Borsäure und Natriumtetraborat-Decahydrat; oder
worin die Borationenquelle Borsäure zusammen mit einer Base ist.

10. Zusammensetzung nach Anspruch 1, worin das modifizierte Polysaccharid ein mit einer flüchtigen Säure vernetztes Polysaccharid ist.

11. Zusammensetzung nach Anspruch 10, worin die flüchtige Säure Salzsäure ist.

12. Zusammensetzung nach einem der Ansprüche 8 und 10, worin die Zusammensetzung einen pH-Wert in dem Bereich von pH 2 bis pH 11 aufweist.

13. Zusammensetzung nach einem der Ansprüche 8 und 11, worin das hydrophile Polysaccharid ein Polysaccharidexsudat ist, das aus der Gruppe von Gewürzen, Sträuchern und Bäumen ausgewählt unter Sterculiaceae, Cochlospermaceae, Rutaceae, Proteaceae, Combretaceae, Rosaceae, Anacardiaceae, Meliaceae, Leguminosae, Rhizophoraceae, Celastraceae, Moringaceae, Sapindaceae, Annonaceae, Burseraceae, Bombacaceae, Arecaceae, Lythraceae, Lecythidaceae, Boraginaceae, Malvaceae, Cactaceae, Bromeliaceae, Guttiferae, Sapotaceae, Pinaceae, Capparidaceae, Araliaceae, Vitaceae, Penaeaceae, Zamiaceae, Cycadaceae, Stangeriaceae, Ebenaceae, Agavaceae, Elaeocarpaceae, Cunoniaceae, Gymnospermeae, Ochnaceae, Rhamnaceae, Euphorbiaceae und Pittosporaceae-Familien abgeleitet ist.

14. Zusammensetzung nach Anspruch 13, worin das Polysaccharidexsudat ausgewählt ist unter *Sterculia urens* und *Bauhinia variegata* Arten.

15. Zusammensetzung nach Anspruch 1,
worin das Nicht-Lösungsmittel ausgewählt ist unter Propylenglycol, Dipropylenglycol, Polyethylenglycol, Butylenglycol, Hexylenglycol, Polyoxyethylen, Polypropylenglycol und Ethylenglycol, worin vorzugsweise das Nicht-Lösungsmittel Propylenglycol ist, oder
worin das Feuchthaltemittel ausgewählt ist unter Glycerol, Sorbitol und Maltitol.

16. Verfahren zum Herstellen einer adhäsiven pharmazeutischen Hydrogelzusammensetzung nach Anspruch 1, wobei das Verfahren die Schritte umfasst:
a) Exponieren des Polysaccharids einem Modifikator;
b) Dispergieren des mindestens einen Polysaccharids in mindestens einem Nicht-Lösungsmittel des Polysaccharids;
c) Vermischen des Lösungsmittels mit dem mindestens einen Feuchthaltemittel;
d) Kombinieren der Polysaccharid-Nicht-Lösungsmittel-Dispersion mit dem Lösungsmittel-Feuchthaltemittel-Gemisch, um ein viskoses Gemisch zu erzeugen; und
e) Dispensieren des viskosen Gemisches.

17. Biologisch adhäsive Hydrogelzusammensetzung zur Verwendung bei einer topischen, systemischen oder transdermalen Verabreichung eines pharmazeutisch aktiven Mittels, umfassend:
a) 10 bis 50-Gew.-% mindestens eines modifizierten hydrophilen Polysaccharids,
b) 20 bis 50-Gew.-% mindestens eines Nicht-Lösungsmittels des Polysaccharids;
c) 10 bis 40-Gew.-% mindestens eines Lösungsmittels des Polysaccharids, und
d) 10 bis 40-Gew.-% mindestens eines Feuchthaltemittels, worin das modifizierte hydrophile Polysaccharid ausgewählt ist aus einem teilweise depolymerisierten Polysaccharid, einem mit Borat-Ionen vernetzten Polysaccharid, und einem mit Säure vernetzten Polysaccharid.

18. Biologisch adhäsive Hydrogelzusammensetzung nach Anspruch 1 zur topischen, systemischen oder transdermalen Verabreichung eines pharmazeutisch aktiven Mittels.

19. Verwendung einer biologisch adhäsiven Hydrogelzusammensetzung nach Anspruch 1 zur topischen Verabreichung eines kosmetischen Mittels.

## Revendications

1. Une composition d'hydrogel bioadhésive comprenant :
a) 10 % à 50 % en poids d'au moins un polysaccharide hydrophile modifié ;
b) 20 % à 50 % en poids d'au moins un non solvant du polysaccharide ;
c) 10 % à 40 % en poids d'au moins un solvant dudit polysaccharide ; et
d) 10 % à 40 % en poids d'au moins un humectant, dans laquelle le polysaccharide hydrophile modifié est sélectionné à partir d'un polysaccharide partiellement dépolymérisé, un polysaccharide réticulé par un ion borate et un polysaccharide réticulé par un acide.

2. La composition selon la revendication 1 comprenant en outre un agent actif sélectionné à partir d'un agent thérapeutique et un agent cosmétique.

3. La composition selon la revendication 1 dans laquelle le polysaccharide modifié est un polymère partiellement dépolymérisé.

4. La composition selon la revendication 3 dans laquelle le polysaccharide est partiellement dépolymérisé au moyen d'un procédé sélectionné à partir de irradiation gamma, radiation UV, exposition à l'ozone, sonification, pression mécanique, chauffage et hydrolyse acide.

5. La composition selon la revendication 4 dans laquelle le polysaccharide est partiellement dépolymérisé par irradiation gamma, de préférence dans laquelle le polysaccharide est partiellement dépolymérisé à l'aide d'ozone et de radiation UV ou dans laquelle le polysaccharide est un exsudat de polysaccharide sélectionné à partir de la famille des sterculiacées et des légumineuses (fabacées), de la famille des herbes, des arbustes et des arbres, de préférence dans laquelle le polysaccharide est sélectionné à partir du groupe consistant en gomme karaya et gomme arabique, plus préférablement dans laquelle le polysaccharide est la gomme karaya ; ou dans laquelle le polymère partiellement dépolymérisé possède un poids moléculaire moyen de 0.1 x 10⁶ à 10⁹ Daltons ; ou dans laquelle le polymère partiellement dépolymérisé possède un poids moléculaire moyen de 0.2 x 10⁶ à 10⁸ Daltons.

6. La composition selon la revendication 3 comprenant une fraction à poids moléculaire élevé et une fraction à bas poids moléculaire d'au moins un polysaccharide partiellement dépolymérisé.

7. La composition selon la revendication 6 dans laquelle la fraction à poids moléculaire élevé possède un poids moléculaire de 2 x 10⁶ à 9 x 10⁶ Daltons et la fraction à bas poids moléculaire possède un poids moléculaire de 0.1 x 10⁶ à 2 x 10⁶ Daltons ; ou dans laquelle la fraction à poids moléculaire élevé et la fraction à bas poids moléculaire comprennent de la gomme karaya partiellement dépolymérisée ; ou dans laquelle la fraction à poids moléculaire élevé comprend de la gomme karaya partiellement dépolymérisée et la fraction à bas poids moléculaire comprend de l'exsudat de Bauhinia variegata.

8. La composition selon la revendication 1 dans laquelle le polysaccharide modifié est un polysaccharide réticulé par un ion borate.

9. La composition selon la revendication 8 dans laquelle la source de ion borate est sélectionnée à partir du groupe consistant en acide borique et borate de sodium décahydrate ; ou dans laquelle la source de ion borate est l'acide borique ensemble avec une base.

10. La composition selon la revendication 1 dans laquelle le polysaccharide modifié est un polysaccharide réticulé au moyen d'un acide volatil.

11. La composition selon la revendication 10 dans laquelle l'acide volatil est l'acide chlorhydrique.

12. La composition selon l'une des revendications 8 et 10 dans laquelle la composition possède un pH dans l'intervalle de pH 2 à pH 11.

13. La composition selon l'une des revendications 8 et 11 dans laquelle le polysaccharide hydrophile est un exsudat de polysaccharide dérivant du groupe des herbes, arbustes et arbres sélectionnés à partir des familles de sterculiacées, cochlospermacées, rutacées, protéacées, combrétacées, rosacées, anacardiacées, méliacées, légumineuses, rhizophoracées, célastracées, moringacées, sapindacées, annonacées,
burséracées, bombacacées, arénacées, lythracées, lécythidacées, borraginacées, malvacées, cactacées, broméliacées, guttiféracées, sapotacées, pinacées, capparidacées, araliacées, vitacées, pénéacées, zamiacées, cycadées, stangériacées, ébénacées, agavacées, élaéocarpacées, cunoniacées, gymnospermées, ochnacées, rhamnacées, euphorbiacées et pittosporacées.

14. La composition selon la revendication 13 dans laquelle l'exsudat de polysaccharide est sélectionné à partir des espèces Sterculia urens et Bauhinia variegata.

15. La composition selon la revendication 1 dans laquelle le non solvant est sélectionné à partir de propylène glycol, dipropylène glycol, polyéthylène glycol, butylène glycol, hexylène glycol, polyoxyéthylène, polypropylène glycol et éthylène glycol, de préférence dans laquelle le non solvant est le propylène glycol ; ou dans laquelle l'humectant est sélectionné à partir de glycérol, sorbitol et mannitol.

16. Un procédé de préparation d'une composition pharmaceutique d'hydrogel adhésive selon la revendication 1, le procédé comprenant les étapes de :
a) exposer le polysaccharide à un agent modifiant ;
b) disperser ledit au moins un polysaccharide dans au moins un non solvant dudit polysaccharide ;
c) mélanger ledit solvant avec le au moins un humectant ;
d) combiner ladite dispersion de polysaccharide-non solvant avec ledit mélange solvant-humectant pour générer un mélange visqueux ; et
e) déposer ledit mélange visqueux.

17. Une composition d'hydrogel bioadhésive pour usage dans une administration topique, systémique or transdermique d'un agent pharmaceutiquement actif comprenant :
a) 10 % à 50 % en poids d'au moins un polysaccharide hydrophile modifié ;
b) 20 % à 50 % en poids d'au moins un non solvant du polysaccharide ;
c) 10 % à 40 % en poids d'au moins un solvant dudit polysaccharide ; et
d) 10 % à 40 % en poids d'au moins un humectant, dans laquelle le polysaccharide hydrophile modifié est sélectionné à partir d'un polysaccharide partiellement dépolymérisé, un polysaccharide réticulé par un ion borate et un polysaccharide réticulé par un acide.

18. Composition d'hydrogel bioadhésive selon la revendication 1 pour l'administration topique, systémique ou transdermique d'un agent pharmaceutiquement actif.

19. Utilisation d'une composition d'hydrogel bioadhésive selon la revendication 1 pour l'administration topique d'un agent cosmétique.
